# EUROPÄISCHE PATENTANMELDUNG

(11) **EP 4 124 283 A1**
(43) Veröffentlichungstag der Anmeldung: **01.02.2023**
(21) Anmeldenummer: 22185947.3
(22) Anmeldetag: 20.07.2022
(51) Int. Cl.: A61B 1/00, A61B 5/107, G06T 7/55, A61B 90/00

(54) **MESSVERFAHREN UND EINE MESSVORRICHTUNG**

(30) Priorität: 27.07.2021 DE 102021119481
(71) Anmelder: Karl Storz SE & Co. KG, 78532 Tuttlingen (DE)
(72) Erfinder: Buschle, Lukas, 78532 Tuttlingen (DE); KÖHLER, Benedikt, 78532 Tuttlingen (DE); Haag, Simon, Tuttlingen (DE); Keuser, Jasmin, Tuttlingen (DE)

(57) **Zusammenfassung**

Die vorliegende Erfindung betrifft ein Messverfahren (50) und eine Messvorrichtung (10), um innerhalb einer Szene (32) eine Länge oder eine Fläche zu ermitteln, die zumindest teilweise durch einen Realstartpunkt (40-2) und einen Realendpunkt (42-2) gekennzeichnet sind, wobei die Messung unter Verwendung von mindestens zwei Bildern (18, 20) erfolgt und es dabei nicht erforderlich ist, dass der Realstartpunkt (40-2) und der Realendpunkt (42-2) in einem der Bilder (18, 20) abgebildet sind.

## Beschreibung

Die vorliegende Erfindung betrifft ein Messverfahren und eine Messvorrichtung.

Optische Visualisierungssysteme wie Mikroskope, Exoskope und Endoskope ermöglichen eine Darstellung eine Szene. Bei der Szene handelt es sich üblicherweise um einen Arbeitsbereich, in dem feinmotorische Arbeiten oder visuelle Überprüfungen durchgeführt werden. Bei medizinischen Eingriffen ist der Arbeitsbereich ein Operationsfeld in einem inneren Bereich des menschlichen Körpers.

Die Endoskopie ist bspw. eine bildgebende Technik, bei der ein Endoskop in einen Hohlraum eingeführt wird. Das Fachpersonal, welches einen solchen Eingriff mit einem Endoskop durchführt, betrachtet das vom Endoskop erfasste Bild auf dem Bildschirm und kann dementsprechend seine Handlungen lenken. Bei medizinischen Eingriffen wird ein Endoskop in den Körper eingeführt, um ein inneres Bild des Körpers zu erfassen und auf dem Bildschirm darzustellen. Solche Eingriffe mit Hilfe der Endoskopie, welche in der Medizin auch als minimal-invasive Chirurgie bezeichnet werden, benötigen in der Regel lediglich deutlich kleinere Schnitte als herkömmliche Verfahren wie z.B. die offene Chirurgie, da eine direkte Sicht auf einen Bereich, an dem der Eingriff durchgeführt wird, nicht erforderlich ist.

Aufgrund der benötigten präzisen Arbeitsweise des Fachpersonals ist es wünschenswert, dass ein genaues Bild des Hohlraums bzw. Bereichs vermittelt wird, an dem die Inspektion oder die Operation durchgeführt wird. In der Regel sind Bilder, die von einem Endoskop auf einem Anzeigegerät wiedergegeben werden, zweidimensional und es ist dem Fachpersonal somit nicht möglich, die Dimensionen und Abmessungen von Strukturen in der abgebildeten Szene einfach und verlässlich zu bestimmen. Dies stellt für die benötigte präzise Arbeitsweise eine Herausforderung dar.

Die zweidimensionale und oftmals vergrößerte Darstellung von Strukturen in einer Szene, die zudem bei einer Weitwinkelaufnahme ohne Rektifizierung verzerrt sein kann, führt vielmehr häufig zu einer falschen Wahrnehmung der Dimensionen und Abmessungen der Struktur in der Szene. In vielen Fällen ist es somit für das Fachpersonal wünschenswert, eine einfache Messfunktion zur Verfügung zu haben. Bei medizinischen Eingriffen kann dies beispielsweise dazu dienen, die Größe einer Kraniotomie, eines Aneurysmas, Tumors oder einer anderen beliebigen anatomischen Struktur intraoperativ zu messen.

Bei medizinischen Eingriffen kann die Abschätzung eines Abstands, der mit der inneren Anatomie eines Patienten während eines minimal-invasiven chirurgischen Eingriffs verbunden ist, darin bestehen, robotermanipulierte chirurgische Instrumente an verschiedenen Positionen innerhalb des Patienten zu positionieren und den Abstand zwischen den Instrumenten auf der Grundlage kinematischer Daten für die Instrumente abzuschätzen.

Es ist auch möglich über die Fokusposition des medizinischen Instruments, wie bspw. des Endoskops, einen Arbeitsabstand zur anatomischen Struktur zu bestimmen, um mit dieser Information reale Abstände im aufgenommenen Bild zu berechnen und anzuzeigen. Dies kann beispielsweise in der Form eines Maßstabs erfolgen.

DE 10 2011 114 146 A1 offenbart beispielsweise ein Verfahren und eine Vorrichtung zum Darstellen eines Objekts, die eine möglichst gute Beurteilung der Dimension des Objekts ermöglichen. Bei dem Objekt handelt es sich beispielsweise um Gewebe und das offenbarte Verfahren kann zur Kontrolle einer Operation des Gewebes verwendet werden.

Trotz einiger vielversprechender Ansätze besteht weiterhin ein Bedarf, dass eine verlässliche Vermessung und Größenbestimmung von Strukturen in der aufgenommenen Szene nicht nur dann möglich ist, wenn ein statisches Bild der Szene aufgenommen wurde, sondern zum Beispiel bereits zeitgleich zur Bewegung des Instruments während der Inspektion oder des Eingriffs. In diesem Fall würde es dem Fachpersonal unter anderem ermöglicht werden, die Größe einer Struktur live während der der Inspektion oder des Eingriffs zu bestimmen. Das Fachpersonal könnte dementsprechend seine Handlungen darauf basierend besser lenken und Schlüsse ziehen, die bisher nicht möglich sind.

In Anbetracht dessen liegt der vorliegenden Erfindung die Aufgabe zugrunde, ein einfaches und verbessertes Messverfahren sowie eine entsprechende Messvorrichtung bereitzustellen, durch welche das Fachpersonal die Größe und Dimension von Strukturen in einer Szene bestimmen kann.

Nach einem Aspekt wird die Aufgabe gelöst durch ein Messverfahren, aufweisend die folgenden Schritte:
Aufnehmen eines ersten Bildes von einer Szene, wobei das Aufnehmen mit einem Bildsensor, vor dem ein optisches System angeordnet ist, erfolgt, wobei in dem ersten Bild eine erste Vielzahl von ersten Bildpunkten eine entsprechende erste Vielzahl von ersten Realpunkten der Szene mittels des Bildsensors abbildet und wobei ein Bildstartpunkt der ersten Vielzahl von ersten Bildpunkten einen Realstartpunkt in der Szene definiert, wobei sich ein Sichtfeld des Bildsensors beim Aufnehmen des ersten Bildes in einer ersten räumlichen Lage relativ zu der Szene befindet;
Ermitteln von ersten Tiefeninformationen, die zumindest für eine erste Teilmenge der ersten Vielzahl von ersten Bildpunkten, einschließlich des Bildstartpunkts, einen jeweiligen Abstand eines von einem ersten Bildpunkt abgebildeten ersten Realpunkts der ersten Vielzahl von ersten Realpunkten zu einer Referenzebene beschreiben;
Aufnehmen eines zweiten Bildes der Szene, wobei das Aufnehmen mit dem Bildsensor erfolgt, wobei in dem zweiten Bild eine zweite Vielzahl von zweiten Bildpunkten eine entsprechende zweite Vielzahl von zweiten Realpunkten der Szene mittels des Bildsensors abbildet und wobei ein Bildendpunkt der zweiten Vielzahl von zweiten Bildpunkten einen Realendpunkt in der Szene definiert, wobei sich das Sichtfeld des Bildsensors beim Aufnehmen des zweiten Bildes in einer zweiten räumlichen Lage relativ zu der Szene befindet, die sich von der ersten räumlichen Lage unterscheidet;
Ermitteln von zweiten Tiefeninformationen, die zumindest für eine zweite Teilmenge der zweiten Vielzahl von zweiten Bildpunkten, einschließlich des Bildendpunkts, einen jeweiligen Abstand eines von einem zweiten Bildpunkt abgebildeten zweiten Realpunkts der zweiten Vielzahl von zweiten Realpunkten zu der Referenzebene beschreiben;
Bestimmen einer Gruppe von Punktepaaren, die jeweils einen ersten Bildpunkt aus der ersten Vielzahl von ersten Bildpunkten und einen zweiten Bildpunkt aus der zweiten Vielzahl von zweiten Bildpunkten enthalten, die miteinander derart korrespondieren, dass der erste und der zweite Bildpunkt eines Punktepaars denselben Realpunkt abbilden;
Bestimmen einer räumlichen Lageänderung des Sichtfelds des Bildsensors auf Basis der Gruppe von Punktepaaren;
Berechnen von Messergebnisinformation basierend auf einer ersten Position des Bildstartpunkts auf dem Bildsensor, einer zweiten Position des Bildendpunkts auf dem Bildsensor, einer Tiefeninformation für den Bildstartpunkt und/oder den Bildendpunkt, den Abbildungseigenschaften des optischen Systems und der räumlichen Lageänderung des Sichtfelds, wobei die Messergebnisinformation eine Länge oder eine Fläche in der Szene ist; und
Ausgeben der Messergebnisinformation.

Das Bestimmen der räumlichen Lageänderung des Sichtfeld des Bildsensors beruht auf der Erkenntnis, dass sich die räumliche Lage des Sichtfelds in drei Freiheitsgraden einer Translation, insbesondere x-Koordinate, γ-Koordinate und z-Koordinate, und in drei Freiheitsgraden einer Rotation, insbesondere α-Winkel, β-Winkel, γ-Winkel, sog. Eulerwinkel oder Kardanwinkel, ändern kann. Auf Grundlage dieser sechs Variablen ist es möglich zu beschreiben, wie vom einem Bezugspunkt des Bildsensors aus ein bestimmter Realpunkt, der in der ersten räumlichen Lage des Sichtfelds mit den Koordinaten (x, y, z) erfasst wird, in der zweiten räumlichen Lage des Sichtfelds dann mit den Koordinaten (x', y', z') erfasst wird. Umgekehrt bedeutet dies, dass sich, sofern sich genügend unabhängige Gleichungen aufstellen lassen, aus erkannten resultierenden Transformationen von Realpunkten pₙ (xₙ, yₙ, zₙ) im ersten Bild nach Bildpunkten p'ₙ (x'ₙ, y'ₙ, z'ₙ) im zweiten Bild die sechs Variablen bestimmen lassen, die die räumliche Lageänderung beschreiben. So wird die räumliche Lageänderung des Sichtfelds des Bildsensors auf Basis der Gruppe von Punktepaaren bestimmt.

Dabei wird insbesondere zumindest näherungsweise angenommen, dass sich die beobachteten Punkte bzw. das beobachtete Objekt selbst nicht bewegen bzw. bewegt. Dafür wird eine reine affine Transformation verwendet: p' = R*p + t, wobei p der Vektor zu einem Realpunkt ist, dessen Koordinaten mit Hilfe des ersten Bilds gewonnen werden, p' der Vektor zu dem entsprechenden transformierten Realpunkt ist, dessen Koordinaten mit Hilfe des zweiten Bilds gewonnen werden, t ein Translationsvektor und R eine Rotationsmatrix ist.

Wenn die räumliche Lageänderung bekannt ist, können die Positionen von Bildstartpunkt und Bildendpunkt, jeweils einschließlich der jeweiligen Tiefeninformation, in ein gemeinsames Bezugssystem umgerechnet werden. Dabei kann bei einer bevorzugten Ausführungsform die Position des Bildstartpunkts, der in der ersten räumlichen Lage des Sichtfelds des Bildsensors aufgenommen wurde, in eine transformierte Position umgerechnet werden, wobei diese transformierte Position angibt, in welcher Position der Bildsensor den Bildstartpunkt aufgenommen hätte und mit welchem Wert dessen Tiefeninformation bestimmt worden wäre, wenn der Bildstartpunkt in der zweiten räumlichen Lage des Sichtfelds des Bildsensors aufgenommen worden wäre. Umgekehrt ist es bei einer weiteren bevorzugten Ausführungsform auch möglich, die Position des Bildendpunkts, der in der zweiten räumlichen Lage des Sichtfelds des Bildsensors aufgenommen wurde, in eine transformierte Position umzurechnen, wobei diese transformierte Position angibt, in welcher Position der Bildsensor den Bildendpunkt aufgenommen hätte und mit welchem Wert dessen Tiefeninformation bestimmt worden wäre, wenn der Bildendpunkt in der ersten räumlichen Lage des Sichtfelds des Bildsensors aufgenommen worden wäre.

Da nun die Bezugspunkte, also der Bildstartpunkt und der Bildendpunkt, in einem gemeinsamen Bezugssystem angegeben sind, können Berechnungen auf Basis der Position der Bezugspunkte durchgeführt werden, also eine Messergebnisinformation in Bezug auf die Realstart- und Realendpunkte berechnet werden. Im einfachsten Fall, wenn die zu vermessende Szene als im Wesentlichen eben und parallel zur Bildebene betrachtet werden kann, ist es ausreichend, die x- und γ-Positionen der Bezugspunkte zu betrachten, also in der Bildebene des optischen Systems. Um nun aus dem x- und γ-Positionen in der Bildebene des optischen Systems tatsächliche Abstände in der Szene zu ermitteln, müssen neben der Tiefeninformation, hier der Abstand der Bildebene des optischen Systems zur Szene, die Abbildungseigenschaften des optischen Systems bekannt sein.

Zu den Abbildungseigenschaften des optischen Systems zählen insbesondere der Öffnungswinkel und der Vergrößerungsfaktor bzw. Zoomfaktor. Sind diese optischen Abbildungseigenschaften des optischen Systems bekannt, so können Bildpunktabstände innerhalb eines Bilds in Realpunktabstände in der Szene umgerechnet werden, wenn zudem auch der Abstand zwischen Bildebene und Ebene der Szene bekannt ist. Der Vergrößerungsfaktor bzw. Zoomfaktor des optischen Systems kann beispielsweise durch die Erfassung eines manuellen Stellelements zur Einstellung der Vergrößerung oder des Zooms, durch die Erfassung einer Motorposition oder Motoransteuerung des Zooms bestimmt werden, von einer angeschlossenen Kamerakontrolleinheit (CCU) automatisch eingestellt oder aus einem Bild ermittelt werden. So kann sich beispielsweise rechnerisch ergeben, dass bei bestimmten Abbildungseigenschaften eines optischen Systems und einer bestimmten Tiefe der Abstand zwischen den Mittelpunkten von zwei benachbarten Bildpunkten des Bildsensors einem Abstand von 0,1mm in der Szene entspricht. Wird also ermittelt, dass Bildstartpunkt und Bildendpunkt im gemeinsamen Bezugssystem einen Abstand von beispielsweise 124 Bildpunkten haben, so ist der entsprechende tatsächliche Abstand in der Szene 124 x 0,1 mm = 12,4mm.

Mit den bekannten Abbildungseigenschaften des optischen Systems, kann unter Berücksichtigung der Tiefeninformation errechnet werden, welche Strecke gemessen in Bildpunkten welcher Strecke in einer Längeneinheit in der Szene entspricht.

Normalerweise wird die zu vermessende Szene nicht als im Wesentlichen eben und parallel zur Bildebene betrachtet werden können. Daher kann zusätzlich die Tiefeninformation des Bildstartpunkts, also ein erster Abstand des Realstartpunkts von der Bildebene, und die Tiefeninformation des Bildendpunkts, also ein zweiter Abstand des Realendpunkts von der Bildebene, berücksichtigt werden. Bevorzugt wird die Tiefeninformation aller oder einer Vielzahl von Bildpunkten fortlaufend ermittelt und damit sogenannte Tiefenkarten errechnet.

Bei einigen bevorzugten Ausführungsformen wird die Tiefeninformation für mindestens einen Bezugspunkt, also den Bildstartpunkt, den Bildendpunkt oder ähnliche weitere Punkte, z.B. einen Bildfolgepunkt, aus der Tiefeninformation von nächstliegenden Punkten gewonnen. Auf diese Weise ist es möglich einem solchen Bezugspunkt eine Tiefeninformation zuzuordnen, selbst wenn diese Tiefeninformation für diesen Bezugspunkt nicht oder nicht hinreichend genau bestimmt werden konnte. So kann diesem Bezugspunkt zum Beispiel die Tiefeninformation des entsprechenden nächstliegenden Punkts zugeordnet werden. Alternativ kann aus mindestens zwei nächstliegenden Punkten ein Mittelwert der Tiefeninformation gebildet und dem Bezugspunkt zugeordnet werden. Werden mehrere nächstliegende Punkte verwendet, kann jeder der nächstliegenden Punkte mit größerer Nähe zum entsprechenden Bezugspunkt höher gewichtet werden. Es sei darauf hingewiesen, dass in gleicher Weise auch allen Bildpunkten, bei denen eine Tiefeninformation nicht oder nicht hinreichend genau bestimmt werden konnte, eine Tiefeninformation zugeordnet werden kann, so dass sich eine zumindest weitestgehend vollständige Tiefenkarte für das bzw. für jedes Bild ergibt.

Der Unterschied zwischen der ersten räumlichen Lage und der zweiten räumlichen Lage, die von der ersten räumlichen Lage verschieden ist, ergibt sich dadurch, dass der Bildsensor oder zumindest das Sichtfeld des Bildsensors translatorisch und/oder rotatorisch relativ zur Szene bewegt wird.

Es ist bevorzugt, wenn einem Benutzer ein Bildzielpunkt angezeigt wird, insbesondere als Overlay auf einem Bildschirm, der ein vom Bildsensor aktuell erfasstes Bild anzeigt, wobei das Bild Teil einer fortlaufenden Abfolge von von dem Bildsensor erfassten Videobildern ist. Dies erleichtert dem Benutzer die korrekte Positionierung des Bildzielpunkts als Bildstart- bzw. Bildendpunkt, wobei beim Aufnehmen des ersten Bildes der Bildzielpunkt den Bildstartpunkt festlegt, und beim Aufnehmen des zweiten Bildes der Bildzielpunkt den Bildendpunkt festlegt. Bei einigen Ausführungsformen können diese Bezugspunkte, also der Bildstartpunkt und der Bildendpunkt, unterschiedlich gewählt werden, indem der Bildzielpunkt zwischen dem Aufnehmen des ersten Bilds und dem Aufnehmen des zweiten Bilds neu positioniert wird. Beispielsweise wird der Bildzielpunkt insbesondere bei Betrachtung des Live-Videobildes der Szene mit Hilfe eines Eingabemittels, z.B. einer Maus oder einer 3D-Maus zur Bedienung mit der Hand oder mit dem Fuß, in Form eines Cursors, Fadenkreuzes oder dergleichen, vom Benutzer an die gewünschte Stelle bewegt, oder beispielsweise mit dem Finger auf einem Touchscreen gesetzt. Er wird also mit einem Realstart- bzw. Realendpunkt in der Szene in Deckung gebracht und so der Bildstart- und der Bildendpunkt ausgewählt, die der Berechnung der Messergebnisinformation zwischen den Realpunkten zugrunde gelegt werden sollen. Die Punkte werden also als Messpunkte ausgewählt. Dabei kann der Bildsensor relativ zur Szene neu positioniert oder ausgerichtet werden. Bei einer bevorzugten Ausgestaltung sind die Bezugspunkte fest vorgegeben, das heißt, es sind vorgegebene Bildpunkte auf dem Bildsensor. Dabei können die Bezugspunkte (Bildstart- und Bildendpunkt) insbesondere identisch sein Dies kann die Handhabung erleichtern, da der Benutzer nun durch Änderung der räumlichen Lage des Bildsensors, insbesondere des Instruments, beispielsweise eines Endoskops, in dem der Bildsensor angeordnet ist, oder durch Anpassung des Sichtfelds des Bildsensors, den Bildzielpunkt in Deckung mit dem gewünschten Realpunkt bringt, der im Bild dargestellt wird. Das Instrument, das den Bildsensor enthält, wird also selbst zum Eingabemittel. Dann wird das jeweilige Bild aufgenommen und der jeweilige Realstart- oder Realendpunkt für die jeweilige Aufnahme festgelegt bzw. definiert, der der Bestimmung der Messergebnisinformation zugrunde liegen soll. Bei einer bevorzugten Ausgestaltung wird der Bildzielpunkt als Bildmitte des Bildsensors gewählt. Bevorzugt erfolgt die Auswahl von Punkten für die Messung, während dem Benutzer ein Video von der Szene in Echtzeit angezeigt wird. Der Benutzer kann so auf einfache und intuitive Weise die gewünschten Punkte in unterschiedlichen Bildern auswählen. Die Auswahl zwischen welchen Realstart- und Realendpunkten die Messergebnisinformation berechnet werden soll erfolgt beispielsweise durch einen Knopfdruck oder ein anderes Auslösen eines Signals an dem Eingabemittel.

Der Bildsensor kann für Licht im sichtbaren Bereich empfindlich sein. Es ist auch möglich, dass der Bildsensor zusätzlich oder alternativ dazu für Licht im Nahinfrarot-Bereich empfindlich ist, was insbesondere eine Fluoreszenzbeobachtung ermöglicht. Bei einigen Ausgestaltungen ist der Bildsensor Element eines Videoendoskops, eines Videoexoskops oder eines Videomikroskops, sowohl für den medizinischen als auch für den nicht-medizinischen oder technischen Einsatz.

Die räumliche Lage, auch Pose genannt, bezeichnet die Kombination von Position und Orientierung eines Objektes. Es können über die räumliche Lage somit drei Rotations- und drei Translationsfreiheitsgrade beschrieben werden.

Die Referenzebene kann vom Fachmann frei gewählt werden, da die Bestimmung der räumlichen Lageänderung des Sichtfelds des Bildsensors auf einer relativen Änderung von Realpunkten beruht, wie sie von dem Bildsensor wahrgenommen wird. So ist es insbesondere möglich, die Bildebene des optischen Systems oder eine zu dieser Bildebene parallele Ebene als Referenzebene zu betrachten.

Bei dem Bildsensor kann es sich insbesondere um einen Monosensor, der genau ein Bild von der Szene erzeugt, oder einen Stereosensor handeln, der zwei verschiedene Bilder von der Szene erzeugt. Der Stereosensor kann eine durchgehende lichtempfindliche Fläche aufweisen, die in einem ersten Bereich ein linkes Bild bzw. Teilbild der Szene und in einem zweiten Bereich ein rechtes Bild bzw. Teilbild der Szene aufnimmt. Der Stereosensor kann auch zwei voneinander getrennte lichtempfindliche Flächen aufweisen, wobei die eine Fläche ein linkes Bild bzw. Teilbild der Szene und die andere Fläche ein rechtes Bild bzw. Teilbild der Szene aufnimmt. Die getrennten lichtempfindlichen Flächen können dabei jeweils in einem von zwei vollständig getrennten Sensoren angeordnet sein.

Die Bestimmung der Punktepaare kann mittels bekannter Verfahren zur Bildregistrierung erfolgen, so zum Beispiel mittels ORB, SIFT oder SURF, siehe zum Beispiel die Veröffentlichung "Image Matching Using SIFT, SURF, BRIEF and ORB: Performance Comparison for Distorted Images" von Ebrahim Karami, Siva Prasad, und Mohamed Shehata, Faculty of Engineering and Applied Sciences, Memorial University, Canada, sowie die darin zitierten Veröffentlichungen.

Der Begriff der räumlichen Lageveränderung des Sichtfelds des Bildsensors soll dahingehend verstanden werden, dass sowohl der Fall eines starren optischen Systems als auch der Fall eines optischen Systems mit veränderbarer Sichtachse oder Blickrichtung, zum Beispiel durch ein Prisma oder verstellbare Spiegel, erfasst sein soll. Bei einem starren optischen System führt eine räumliche Lageveränderung des fest dazu angeordneten Bildsensors auch unmittelbar zu einer räumlichen Lageveränderung des Sichtfelds des Bildsensors. Dann bedeutet der Begriff der räumlichen Lageveränderung des Sichtfelds des Bildsensors eine räumliche Lageveränderung des Bildsensors. Bei einem optischen System mit veränderbarer Sichtachse kann das Sichtfeld verändert werden, ohne dass sich die räumliche Lage des Bildsensors verändern muss. So kann beispielsweise durch die Verlagerung eines Prismas oder eines Spiegels entlang des Strahlengangs die räumliche Lage des Sichtfelds verändert werden, ohne dass sich die die räumliche Lage des Bildsensors verändert.

In der Praxis hat sich gezeigt, dass sich bei einer FullHD-Auflösung (1920×1080 Bildpunkte) im ersten Bild und im zweiten Bild viele Hunderte oder Tausende markante Stellen wie Kanten oder kontrastreiche Bereiche automatisch identifizieren lassen. Mittels eines Vergleichs des ersten und zweiten Bilds lassen sich beispielsweise 100 Stellen im ersten Bild etwa 100 Stellen im zweiten Bild zuordnen. Berücksichtigt man ferner, dass insbesondere voneinander entfernte Bildpunkte eine hohe Wahrscheinlichkeit einer linearen Unabhängigkeit bzgl. der zu bestimmenden räumlichen Lageänderung versprechen, darf man üblicherweise rund 30 bis 50 Punktepaare erwarten, die eine gute Bestimmung, zumindest näherungsweise, der sechs Variablen erlauben. Entsprechende optimierende Algorithmen, um die Rotation und Translation zu schätzen, sind dem Fachmann bekannt, z.B. RANSAC-Algorithmen.

Bei der Bestimmung der räumlichen Lageänderung kann man sich bevorzugt Vorkenntnisse zu Nutze machen, z.B. wenn ein Freiheitsgrad aufgrund einer äußeren Randbedingung fixiert ist und/oder ein erster Freiheitsgrad in einer definierten Abhängigkeit zu mindestens einem zweiten Freiheitsgrad steht. So kann es für die Bewegung des Instruments bestimmte Randbedingungen geben, z.B. wenn der Bildsensor Teil eines starren Instruments ist, das in einem Trokar steckt. Dann kann der Bildsensor nur entlang des Trokarschafts verschoben werden, und, da der Trokar durch eine kleine Öffnung in den zu untersuchenden Hohlraum eingeführt wird, die somit einen Schwenkpunkt bzw. Pivot-Punkt für den Trokar darstellt, ergibt ein Verschwenken des Bildsensors auch unmittelbar eine definierte Translation. Es können daher bekannte Fixpunkte oder Einschränkungen der Freiheitsgrade berücksichtigt werden, um die Berechnung der Bewegung zu verbessern, zu vereinfachen oder auf Plausibilität zu prüfen. Alternativ oder zusätzlich können Daten von einem Bewegungssensor verwendet werden, wie nachfolgend noch erläutert wird.

Darüber hinaus kann die Information über die bestimmte räumliche Lageänderung verwendet werden, um die fortlaufend erstellten Tiefenkarten der Szene zu verbessern oder auf Plausibilität zu prüfen. So können Werte für verschiedene Realpunkte der im Verfahren bisher erstellten Tiefenkarten unter Verwendung der ermittelten Rotation und Translation in das jeweils aktuelle Bezugssystem überführt werden. Aus mehreren solchen Tiefenwerten für einen Realpunkt können Mittelwerte gebildet und Unsicherheiten, z.B. die Standardabweichung, bestimmt werden. Auf diese Weise ist eine Überprüfung der Qualität der ermittelten Tiefenwerte möglich. Auch können im aktuellen Bild, also der aktuellen Tiefenkarte, fehlende Tiefenwerte so bei Bedarf aus vorigen Werten abgeleitet und automatisch ergänzt werden.

Vorwissen, wie z.B. über ein Pivot-Punkt oder Messdaten von einem Inertialsensor verändern die oben beschriebene affine Transformation. So reduzieren sich z.B. bei einem Pivot-Punkt die möglichen Freiheitsgrade der Bewegung des optischen Visualisierungssystems bzw. des Sichtfelds des Bildsensors von sechs auf vier, da es nun nur noch in der linksrechts, oben-unten, rein-raus-Richtung und um die eigene Achse gedreht werden kann. Dadurch müssen weniger Parameter durch die RANSAC-Methoden ermittelt werden, sodass das Ergebnis stabiler wird. Bei einer Ausführungsform werden die Messdaten von einem Inertialsensor verwendet, um die Rotationsmatrix zu bestimmen. Damit müssen dann nur noch die drei Freiheitgrade der Translationen bestimmt werden. Bei einer weiteren Ausführungsform benutzt man die Inertialsensordaten als Initialisierungswert für die Rotationsmatrix bei einer iterativen Suche nach den Parametern der Bewegung.

Ein Aspekt des offenbarten Messverfahrens basiert auf der Idee, dass eine einfache Messfunktion zur Größen- und Dimensionsbestimmung in einer Szene, insbesondere mit einer anatomischen Struktur, durch eine rein optische Erfassung der Szene möglich ist, sodass auf Navigationssysteme und weitere Sensoren, wie beispielsweise Lagesensoren, verzichtet werden kann.

Dies kann dadurch erreicht werden, dass mit Hilfe des Bildsensors ein erstes Bild und ein zweites Bild der Szene, insbesondere mit der anatomischen Struktur, aufgenommen werden. Das erste Bild und das zweite Bild werden hierbei aus unterschiedlichen räumlichen Lagen des Sichtfelds des Bildsensors aufgenommen. Dies bedeutet, dass sich die Orientierung, die Lage, der Abstand und/oder die Richtung aus der der Bildsensor Bilder der Szene aufnimmt zwischen der Aufnahme des ersten Bildes und des zweiten Bildes ändert. Ferner erfolgt die Aufnahme der Bilder durch ein und denselben Bildsensor, der, wie zuvor erläutert, zwei voneinander getrennte lichtempfindliche Flächen und Leiterplatten aufweisen kann. Bevorzugt ist der Bildsensor Teil eines Stereovideoendoskops mit verbauten, separaten, distalen oder proximalen lichtempfindlichen Flächen. Alternativ kann der Bildsensor Teil eines Mikroskops oder Exoskops sein.

Die Messung findet nicht, wie bereits aus dem Stand der Technik bekannt, in einem statischen Bild statt, in dem zwei Messpunkte automatisch oder durch einen Benutzer ausgewählt werden, sondern der Bildsensor, zumindest das Sichtfeld des Bildsensors, wird manuell oder automatisch verlagert, damit von zwei unterschiedlichen räumlichen Lagen zwei verschiedene Bilder aufgenommen werden können in denen Bildstart- und Bildendpunkte bzw. zugehörige Realstart- und Realendpunkte für die Ermittlung einer Messergebnisinformation definiert werden. Die Bilder sind insbesondere Teil eines in Echtzeit oder nahezu in Echtzeit dem Benutzer angezeigten Videobildes der Szene. Ein solches Videobild kann beispielsweise eine Auflösung von 4K aufweisen und mit wenigstens 30 fps (Frames pro Sekunde) dargestellt werden. Somit ist eine robuste Bestimmung des tatsächlichen Abstands zweier zu unterschiedlichen Zeitpunkten und in unterschiedlichen Bildern aufgenommener Realpunkte in Echtzeit möglich, auch wenn sich die Position des Bildsensors dabei ändert.

Insbesondere ermöglicht das vorliegende Verfahren die Ermittlung von Messinformation zwischen Realpunkten, die nicht gleichzeitig im Sichtfeld des Bildsensors liegen. Bildstartund Bildendpunkt können also so gewählt werden, dass die Vermessung zwischen Punkten erfolgt, die nicht gleichzeitig für den Anwender einsehbar sind, zum Beispiel weil die zu vermessende Struktur zu groß ist. Hierfür können bei Bedarf sowohl Tiefeninformationen für eine Mehrzahl von Punkten als auch Rotation und Translation des Bildsensors fortlaufend aus Punktepaaren in verschiedenen Bildern ermittelt werden.

Wie erläutert, findet das Bestimmen einer räumlichen Lageänderung des Sichtfelds des Bildsensors auf Basis der Gruppe von Punktepaaren statt, die aus dem ersten und dem zweiten Bild gewonnen werden. Die räumliche Lageänderung kann aber zudem auf Basis weiterer Gruppen von Punktepaaren ermittelt werden, wie nachfolgend noch anhand einer bevorzugten Ausgestaltung erläutert wird.

Es versteht sich, dass das Messverfahren nicht nur für intraoperative Untersuchungen geeignet ist, sondern sich ebenso beispielsweise für die Untersuchung des Mundraums eines Patienten eignet. Ferner sind oberflächliche medizinische Untersuchungen möglich, in denen eine einfache Bestimmung von Längen oder Flächen wünschenswert ist.

Bei einer bevorzugten Ausgestaltung weist das Messverfahren ferner die folgenden Schritte auf:
Aufnehmen eines dritten Bildes der Szene, wobei das Aufnehmen mit dem Bildsensor erfolgt, in dem dritten Bild eine dritte Vielzahl von dritten Bildpunkten eine entsprechende dritte Vielzahl von dritten Realpunkten der Szene mittels des Bildsensors abbildet und ein Bildfolgepunkt der dritten Vielzahl von dritten Bildpunkten einen Realfolgepunkt in der Szene definiert, wobei sich das Sichtfeld des Bildsensors beim Aufnehmen des dritten Bildes in einer dritten räumlichen Lage relativ zu der Szene befindet, die sich von der ersten und der zweiten räumlichen Lage unterscheidet;
Ermitteln von dritten Tiefeninformationen, die zumindest für eine dritte Teilmenge der dritten Vielzahl von dritten Bildpunkten, einschließlich des Bildfolgepunkts, einen jeweiligen Abstand eines von einem dritten Bildpunkt abgebildeten dritten Realpunkts der dritten Vielzahl von dritten Realpunkten zu der Referenzebene beschreiben;
Bestimmen einer weiteren Gruppe von Punktepaaren, die jeweils einen zweiten Bildpunkt aus der zweiten Vielzahl von zweiten Bildpunkten und einen dritten Bildpunkt aus der dritten Vielzahl von dritten Bildpunkten enthalten, die miteinander derart korrespondieren, dass der zweite und der dritte Bildpunkt eines Punktepaars denselben Realpunkt abbilden;
Bestimmen einer weiteren räumlichen Lageänderung des Sichtfelds des Bildsensors auf Basis der weiteren Gruppe von Punktepaaren;
wobei das Berechnen der Messergebnisinformation ferner auf einer dritten Position des Bildfolgepunkts auf dem Bildsensor, den dritten Tiefeninformationen und der weiteren räumlichen Lageänderung des Sichtfelds basiert oder eine weitere Messergebnisinformation basierend auf einer dritten Position des Bildfolgepunkts auf dem Bildsensor, den dritten Tiefeninformationen und der weiteren räumlichen Lageänderung des Sichtfelds berechnet wird.

Diese Ausgestaltung ermöglicht es auch weitere Bezugspunkte zu erfassen, die für die Bestimmung einer Länge, zum Beispiel eines mehrteiligen Pfads, oder einer Fläche, zum Beispiel eines Polygons, berücksichtigt werden sollen. Es ist auch möglich, dass dem genannten, ersten Realfolgepunkt und Bildfolgepunkt ein zweiter Realfolgepunkt und Bildfolgepunkt folgt, dem wiederum ein dritter Realfolgepunkt und Bildfolgepunkt folgen kann, usw. Es wird dabei zwischen aufeinanderfolgenden Realfolgepunkten bzw. Bildfolgepunkten, also einem n-ten und einem (n+1)-ten Realfolgepunkt bzw. Bildfolgepunkt eine Gruppe von Punktepaaren mit korrespondierenden Punkten ermittelt, woraus wiederum eine jeweilige n-te räumliche Lageänderung des Sichtfelds des Bildsensors auf Basis der Gruppe von Punktepaaren ermittelt wird. Somit können auch komplexe Pfade und Polygone erfasst werden. Bei einer bevorzugten Ausführungsform können der Realfolgepunkt bzw. die Realfolgepunkte vom Benutzer durch eine Benutzeraktion ausgewählt werden, wie es im Weiteren noch genauer erläutert wird. Bei einer anderen bevorzugten Ausführungsform werden die Realfolgepunkte fortlaufend mit jeder räumlichen Lageänderung des Sichtfelds des Bildsensors erfasst, so dass bereits durch das Führen des Instruments, in das der Bildsensor integriert ist, eine Länge erfasst und ermittelt werden kann, zum Beispiel wenn ein Pfad abgefahren wird.

Bei einer bevorzugten Ausgestaltung werden vor dem Aufnehmen des zweiten oder des dritten Bildes folgende Schritte ausgeführt:
Aufnehmen mindestens eines Zwischenbilds, so dass eine Sequenz beginnend mit dem ersten Bild, fortgeführt über das mindestens eine Zwischenbild und endend mit dem zweiten oder dem dritten Bild gebildet wird, wobei in jedem Zwischenbild eine weitere Vielzahl von weiteren Bildpunkten eine entsprechende weitere Vielzahl von weiteren Realpunkten der Szene mittels des Bildsensors abbildet und wobei sich das Sichtfeld des Bildsensors beim Aufnehmen des Zwischenbildes in einer weiteren räumlichen Lage relativ zu der Szene befindet, die sich von der innerhalb der Sequenz vorherigen räumlichen Lage unterscheidet;
nach dem Aufnehmen eines Zwischenbilds von dem mindestens einen Zwischenbild:
   Ermitteln von weiteren Tiefeninformationen, die zumindest für eine weitere Teilmenge der weiteren Vielzahl von weiteren Bildpunkten einen jeweiligen Abstand eines von einem weiteren Bildpunkt abgebildeten weiteren Realpunkts der weiteren Vielzahl von weiteren Realpunkten zu der Referenzebene beschreiben;
   Bestimmen einer Zwischengruppe von Punktepaaren, die jeweils einen ersten Sequenzbildpunkt aus der weiteren Vielzahl von weiteren Bildpunkten in dem Zwischenbild und einen zweiten Sequenzbildpunkt in einem vorherigen Bild aus der Sequenz enthalten, die miteinander derart korrespondieren, dass der erste und der zweite Sequenzbildpunkt eines Punktepaars einen selben Realpunkt abbilden;
wobei das Bestimmen der räumlichen Lageänderung oder der weiteren räumlichen Lageänderung zudem auf Basis der mindestens einen weiteren Zwischengruppe von Punktepaaren erfolgt.

Wie zuvor erläutert, ist es möglich, die räumliche Lageänderung aus den Informationen zum ersten Bild und zum zweiten Bild zu ermitteln, ohne dass der Realstartpunkt und der Realendpunkt gleichzeitig in einem dieser beiden Bilder abgebildet sein müssen. Entsprechend kann die weitere räumliche Lageänderung aus den Informationen zum zweiten Bild und zum dritten Bild ermittelt werden, ohne dass der Realendpunkt und der Realfolgepunkt gleichzeitig in einem dieser beiden Bilder abgebildet sein müssen.

Die Lageänderung kann grundsätzlich aber auch so groß sein, dass die Bestimmung der Gruppe von Punktepaaren bzw. der weiteren Gruppe von Punktepaaren schwierig oder unmöglich wird. Dies berücksichtigt die bevorzugte Ausgestaltung, indem während der Veränderung der räumlichen Lage mindestens ein Zwischenbild und bevorzugt mehrere Zwischenbilder angefertigt werden. Es wird so eine Sequenz von Bildern gebildet.

Anhand des Verarbeitung eines Bilds aus der Sequenz und eines vorherigen Bilds der Sequenz kann eine Lageänderung ermittelt werden, die zwischen der Aufnahme des Bilds und des vorherigen Bilds stattgefunden hat. Diese Verarbeitung findet prinzipiell genauso statt wie bei der Verarbeitung von erstem und zweitem Bild bzw. von zweitem und drittem Bild. Es ist somit möglich mit Bildern aus der Sequenz Segmente der Lageänderung zu ermitteln und so aus den Segmenten die gesamte Lageänderung zwischen erstem und zweitem Bild bzw. zweitem und drittem Bild zu ermitteln.

Diese Ausgestaltung ermöglicht eine genaue Bestimmung der räumlichen Lageänderung bzw. der weiteren räumlichen Lageänderung auch über größere Distanzen.

Bei einer weiteren bevorzugten Ausgestaltung wird zumindest ein Teil der ersten und zweiten Tiefeninformationen aus einer Stereoinformation gewonnen.

Dabei können verschiedene bekannte Verfahren eingesetzt werden, wie sie beispielsweise in der Bachelorarbeit von Christian Blank mit dem Titel "Generierung von Tiefenbildern mittels Stereoskopie", eingereicht am 13. September 2013, beschrieben und im Literaturverzeichnis zitiert werden. Bei einigen Ausführungsformen wird mittels eines Stereoinstruments das Stereobild der Szene erzeugt, genauer, ein linkes Bild bzw. Teilbild der Szene und ein rechtes Bild bzw. Teilbild der Szene erzeugt, und aus der Disparität zwischen den beiden Bildern des Stereobildes und weiteren Informationen fortlaufend die Tiefeninformationen für viele Bildpunkte aus dem Stereobild bestimmt. Korrespondierende Punkte in dem linken und dem rechten Bild können insbesondere durch Semi-Global-(Block-)Matching bestimmt werden, wie es in der Veröffentlichung "Accurate and efficient stereo processing by semi-global matching and mutual information", Heiko Hirschmüller, IEEE Conference on Computer Vision and Pattern Recognition, 2005, oder in der Veröffentlichung " Semi-Global Matching: Motivation, Development and Applications", Heiko Hirschmüller, Photogrammetric Week, September 2011 beschrieben ist. Daraus erhält man Tiefeninformationen für viele Bildpunkte, insbesondere eine Tiefenkarte. Ein Tiefensensor, der auf einer Laufzeitmessung ("Time of flight (ToF)") basiert, ist dabei nicht notwendig. Es wird darauf hingewiesen, dass unter den Begriffen Stereoskopie und Stereobild auch Pseudostereoskopie und Pseudostereobild fallen. Die Technik der Pseudostereoskopie verwendet aufeinanderfolgende Bilder von einem Instrument, das keine Stereosensoren hat.

Die Tiefeninformation kann bei einer Ausführungsform mit folgender Formel bestimmt werden: Tiefe (in einer Längeneinheit) = Stereobasis (in der Längeneinheit) * Brennweite (in Pixeln) / Disparität (in Pixeln). Dies wird pixelweise berechnet. Die Ermittlung der Brennweite in Pixeln findet gemäß bekannter Verfahren statt. Sie kann insbesondere anhand eines definierten Schachbrettmusters in einer definierten Entfernung bestimmt werden. Aus der Tiefe und der Winkelinformation wird dann auch die x- und γ-Komponente berechnet, wobei die xund γ-Komponente in einer Längeneinheit sein kann. Die Winkelinformation kann dabei aus der Kalibrierung des optischen Systems gewonnen werden, z.B. unter Zuhilfenahme eines Schachbretts.

Bei einer Ausführungsform fließen Kameraparameter in eine anfangs bestimmte Kalibrierung des Messverfahrens ein. Diese verknüpft u.a. die Disparität mit der Tiefe. Die Kalibrierung wird dynamisch an die Einstellungen der Kamera angepasst. Eine Vergrößerung oder ein digitaler Zoom lässt sich bei der Berechnung der Kalibrierung berücksichtigen. Beispielsweise bedeutet eine Vergrößerung um den Faktor zwei, dass sich die Disparität bei konstanter Tiefe verdoppelt.

Eine Besonderheit dieser Ausgestaltung ist zudem, dass der Bildstartpunkt, der Bildendpunkt und evtl. weitere entsprechende Bildpunkte bei Verwendung eines Stereoinstruments, das einen Stereosensor aufweist, auf einem 3D-Bildschirm dargestellt werden können. Dazu wird für jeden Bildpunkt die zugehörige Disparität, also der Versatz der korrespondierenden Bildpunkte auf dem linken und dem rechten Bild bzw. Teilbild, ausgelesen und die Positionen der gesetzten Punkte, insbesondere des Bildstartpunkts und des Bildendpunkts, und evtl. dargestellten Messlinien im ersten Bild um die entsprechende Pixelanzahl im Vergleich zum zweiten Bild verschoben. Es wird also eine Disparität auch für die gesetzten und beispielsweise per Overlay dargestellten Punkte und evtl. Messlinien und -flächen erzeugt, beispielsweise aber auch für den Bildzielpunkt, so dass diese für den Beobachter auf einem 3D-Bildschirm in der richtigen Entfernung erscheinen. Die Punkte und Linien können entweder in einer Ebene dargestellt oder so eingeblendet werden, dass sie auf der Oberfläche der beobachteten Struktur bzw. Szene zu liegen scheinen. Messlinien und -punkte können daher entweder vordergründig in einer Ebene mit gleicher Tiefe oder in der tatsächlichen Tiefe der ausgewählten Strukturen in der dargestellten Szene angezeigt werden. Insbesondere können Messlinien zwischen Bildstart- und Bildendpunkten für den Betrachter in die Tiefe entlang von Konturen abgebildeter Strukturen verlaufen. Bei dem vorliegenden Verfahren kann ein als Realstartpunkt oder Realendpunkt definierter Realpunkt als Messpunkt, beispielsweise per Overlay über das aktuelle Videobild, markiert werden und bei einer Änderung der Position und/oder Lage des Bildsensors kann die Markierung so mitgeführt werden, dass der Messpunkt trotz der veränderten Ansicht weiterhin im Bild dem Realpunkt zugeordnet dargestellt wird. Der Messpunkt haftet sozusagen an der Szene an der richtigen Position und in der richtigen Tiefe. Ebenso kann eine zwischen zwei Punkten dargestellte Messlinie bei einer Bewegung des Bildsensors dargestellt werden.

Bei dem vorliegenden Verfahren werden hierfür insbesondere vor der Bestimmung der Tiefeninformation, d.h. der Disparitäten und der Tiefenkarte, das linke und das rechte Bild rektifiziert. Rektifizierung heißt, Verzerrungseffekte des optischen Systems und eine mögliche rotierte Anordnung der Bildsensoren zueinander werden in den Bildern rechnerisch korrigiert und entfernt und die Bilder parallel zueinander ausgerichtet. Danach werden wie zuvor beschrieben die Disparitätswerte für verschiedene Bildpunkte und damit die Tiefeninformationen bzw. die Tiefenkarte ermittelt. Der Bildstartpunkt oder Bildendpunkt wird, z.B. vom Anwender, in einem nicht rektifizierten Teilbild, beispielsweise dem linken Bild, insbesondere mit dem Bildzielpunkt ausgewählt und dann seine Koordinaten in die rektifizierte Geometrie übertragen. Danach kann der Disparitätswert für den betreffenden Punkt ausgelesen werden. Der betreffende Messpunkt wird nun im zugehörigen rechten Bild um die ausgelesene Disparität in Pixeln verschoben und wieder von der rektifizierten in die normale Bildgeometrie zurücktransformiert und im Bild eingeblendet. So wird der betreffende Punkt im Stereobild scheinbar in der passenden Entfernung, beispielweise auf der Oberfläche einer Struktur, dargestellt. Ein Punkt erscheint in einer größeren Tiefe, wenn die Disparität verkleinert wird und in einer geringeren Tiefe, wenn die Disparität vergrößert wird.

Sollen stattdessen alle Bildstart- und Bildendpunkte in einer Ebene mit gleicher Tiefe dargestellt werden, können alle Punkte auf die maximal vorkommende Disparität eingestellt werden und erscheinen dann im Vordergrund vor der betrachteten Struktur. Bei Bedarf ist jedoch auch eine Darstellung der Messpunkte und -linien in einem 2D-Bild möglich.

Analog kann für eingeblendete Strecken, Zahlenwerte, Flächen etc. vorgegangen werden, sodass diese mit der richtigen Tiefe im 3D-Bild eingeblendet werden.

Es sei darauf hingewiesen, dass die allgemeinen Kenntnisse und Verfahren zur Stereoskopie, wie sie beispielsweise in "Stereoskope HD Produktionen - Messungen statt Mythen", Herausgeber Nikolaus Hottong, Schriftenreihe Fakultät Digitale Medien der HFU Furtwangen University, Dezember 2016, ISBN 3-9810384-9-5, beschrieben und referenziert werden, als bekannt vorausgesetzt werden und hier nicht weiter erläutert werden.

Bei einer weiteren bevorzugten Ausgestaltung weist die Messergebnisinformation einen Abstand zwischen dem Realstartpunkt und dem Realendpunkt auf.

Wie zuvor erläutert können aufgrund der Positionen der Bezugspunkte, also des Bildstartpunkts und des Bildendpunkts, und der jeweiligen Tiefeninformationen räumliche Koordinaten der Bezugspunkte bestimmt werden. Dies erlaubt es mit der üblichen euklidischen Abstandsformel den Abstand zwischen dem Realstartpunkt und dem Realendpunkt zu berechnen.

Bei einer weiteren bevorzugten Ausgestaltung weist die Messergebnisinformation eine Strecke entlang einer Kontur zwischen dem Realstartpunkt und dem Realendpunkt auf.

Bei dieser Ausgestaltung wird angenommen, dass sich anhand einer Bildverarbeitung eine Kontur identifizieren lässt, die zumindest in der Nähe des Realstartpunkts beginnt und zumindest in der Nähe des Realendpunkts endet. Die Strecke kann dann durch Stützpunkte entlang der identifizierten Kontur ermittelt werden, wobei die Stützpunkte, falls nötig, in das Bezugssystem der Bezugspunkte transformiert werden.

Bei einer weiteren bevorzugten Ausgestaltung weist die Messergebnisinformation eine Fläche auf, deren Begrenzung unter Berücksichtigung des Realstartpunkts und des Realendpunkts ermittelt wird.

Bei dieser Ausgestaltung wird angenommen, dass durch den Realstartpunkt und den Realendpunkt eine geschlossene Kontur bestimmt wird, zum Beispiel ein Kreis durch Mittelpunkt und Radius oder durch zwei Kreisbogenpunkte oder ein Rechteck durch zwei gegenüberliegende Ecken. Die Fläche kann, falls die Szene als im Wesentlichen flach angesehen werden kann, einfach mit Unterdrückung der Tiefeninformation im Zweidimensionalen berechnet werden. Soll auch die Tiefeninformation verarbeitet werden, so kann die Kontur in Primitive zerlegt werden, deren Flächen dann zu einer Gesamtfläche addiert werden. Unter Verwendung von drei Punkten, insbesondere Realstartpunkt, Realendpunkt und Realfolgepunkt, kann beispielsweise die Fläche eines so aufgespannten Dreiecks berechnet werden. Stehen mehrere Realfolgepunkte zur Verfügung, lässt sich auch die Fläche von Mehrecken berechnen.

Bei einer weiteren bevorzugten Ausgestaltung wird zumindest ein Teil der ersten und zweiten Tiefeninformationen aus einer Laufzeitinformation gewonnen, die der Bildsensor oder ein Tiefenbildsensor bereitstellt.

Bei dieser Ausgestaltung wird zumindest ein Teil der Tiefeninformationen durch den Bildsensor oder einen Tiefenbildsensor, der die Szene ebenfalls zumindest bezüglich des Realstartpunkts und des Realendpunkts erfasst, ermittelt. So können Tiefeninformationen des ersten aufgenommenen Bildes und des zweiten aufgenommenen Bildes durch Aufnahme eines Tiefenbilds des Sichtfelds des Bilds bestimmt werden. Hierbei sind unterschiedliche Arten von Abstands- bzw. Tiefenmessungen möglich, um die Tiefeninformationen zu ermitteln. Beispiele sind Ultraschallmessungen oder Laserabstandsmessungen (LIDAR). Zudem kann es sich bei dem Tiefenbildsensor um eine Time-of-Flight(ToF)-Kamera handeln. In diesem Fall werden mit einer Beleuchtungseinheit, die vorzugsweise eine oder mehrere LEDs oder Laserdioden aufweist, und welche moduliert angesteuert wird, kurze Lichtpulse ausgesendet, die anschließend von der Szene reflektiert werden. Eine Optik empfängt das reflektierte Licht und bildet die aufgenommene Szene ab. Da ein Tiefenbildsensor in aller Regel über eine geringere Auflösung verfügt als ein allgemeiner Bildsensor bzw. Weißlichtbildsensor, können bei einer Ausführungsform zwischen den vermessenen Punkten des Tiefenbilds weiteren Punkten durch Interpolation eine jeweilige Tiefeninformation zugeordnet werden.

Bei einer weiteren bevorzugten Ausgestaltung wird zumindest ein Teil der ersten und zweiten Tiefeninformationen aus einer Fokusinformation des optischen Systems gewonnen.

Solche optischen Systeme können einen Fokus mit einer fixen Fokuslänge haben. Da der Benutzer den Abstand des Bildsensors zur Szene so wählen wird, dass die Szene zumindest in etwa scharf dargestellt wird, kann die Fokuslänge bei der Ermittlung der ersten und/oder zweiten Tiefeninformation herangezogen werden oder als die erste und/oder zweite Tiefeninformation verwendet werden. Dies kann für einen Teil oder für alle Bildpunkte der ersten und/oder zweiten Teilmenge erfolgen. Wenn das optische System über eine manuelle Stelleinheit zum Verändern der Fokuslänge verfügt, kann durch eine Positionsdetektierung der Stelleinheit die aktuelle Fokuslänge ermittelt werden. Wenn das optische System über eine motorische Stelleinheit zum Verändern der Fokuslänge verfügt, kann durch eine Positionsdetektierung des Motors der Stelleinheit die aktuelle Fokuslänge ermittelt werden.

Bei einer bevorzugten Ausgestaltung erfolgt das Definieren des Realstart- und des Realendpunktes durch eine Benutzeraktion.

Diese Ausgestaltung bietet eine besonders einfache und intuitive Bedienung. Dabei wird dem Benutzer auf einem Bildschirm das Bild vom Bildsensor, insbesondere als fortlaufende Abfolge von Videobildern, z.B. 25, 30, 50 oder 60 Bilder pro Sekunde, mit einem visuell hervorgehobenen Bildzielpunkt, z.B. einem Fadenkreuz, dargestellt. Der Benutzer bringt beispielsweise den Bildzielpunkt mit einem gewünschten Realpunkt, der in dem Bild des Bildsensors dargestellt wird, zur Deckung oder wählt anderweitig wie zuvor beschrieben einen Bildstartpunkt aus und löst durch eine Benutzeraktion, insbesondere mittels eines Knopfdrucks, einer Berührung, eines Tastendrucks, einer Kopf- oder Handgeste oder einem Sprachbefehl, die Definition, also die Festlegung, des betreffenden Realpunkts als Realstartpunkt aus. Das Auswählen und Festlegen des Punktes kann in einer einzigen Benutzeraktion zusammengefasst sein. Das Definieren umfasst beispielsweise das Speichern der Koordinaten des Realpunkts, oder des zugehörigen Bildpunkts und einer Tiefeninformation, in einem Speicher und/oder das Markieren des ausgewählten Punkts im Bild beispielsweise mit Hilfe eines graphischen Overlays in Form eines Messpunkts. Der Benutzer wählt danach wie zuvor beschrieben einen weiteren gewünschten Realpunkt, der in einem weiteren Bild des Bildsensors dargestellt wird, und löst durch eine weitere Benutzeraktion die Definition des weiteren Realpunkts als Realendpunkt aus. Dies kann wie beim Realstartpunkt erfolgen.

Bei einer weiteren bevorzugten Ausgestaltung wird die räumliche Lageänderung des Sichtfelds des Bildsensors unter Berücksichtigung von Bewegungsdaten bestimmt, die von einem Bewegungssensor aufgenommen werden, der relativ zum Bildsensor ortsfest angeordnet ist.

Der Bewegungssensor ist vorzugsweise ein Beschleunigungssensor, der zu Beginn kalibriert wird und Informationen über aus wirkenden Kräften resultierende Beschleunigungen in x-, y-, und z-Richtung liefert. Der Beschleunigungssensor registriert die Bewegungen und durch zweifache Integration der Beschleunigung erhält man einen quantitativen Wert für die Bewegung des Beschleunigungssensors. Die Lage des Sensors gegenüber der Gravitationsachse kann z.B. in Winkeln angegeben werden. Die Verlässlichkeit dieser Messung kann ferner dadurch erhöht werden, dass zusammen mit dem im Beschleunigungssensor verbauten Gyroskop weitere Sensoren verbaut werden, wie z.B. ein Magnetometer. Etwaige auftretende Fehler in der Bestimmung der Bewegungsdaten können durch entsprechende Filterung, wie z.B. durch einen Kalman-Filter oder einen Hochpassfilter, ausgeglichen werden. Bei einigen Ausgestaltungen ist der Bewegungssensor in dem Bildsensor integriert oder am Bildsensor angeordnet.

Die Bewegungsdaten können bevorzugt dafür verwendet werden, Unsicherheiten oder unplausible Werte bei der Ermittlung der räumlichen Lageänderung auf Basis der Gruppe von Punktepaaren zumindest teilweise zu korrigieren oder kompensieren. Deuten ein Wert oder mehrere Werte der ermittelten räumlichen Lageänderung darauf hin, dass die räumliche Lageänderung unzutreffend ermittelt wurde, so können die Bewegungsdaten Werte der räumlichen Lageänderung ersetzen. Bei Unsicherheiten, zum Beispiel wenn verschiedene Teilgruppen von Punktepaaren, die für eine Ermittlung der räumlichen Lageänderung herangezogen werden, zu signifikant unterschiedlichen ermittelten räumlichen Lageänderungen führen, können die Bewegungsdaten gewichtet berücksichtigt werden, sodass das Bestimmen der räumlichen Lageänderung des Sichtfelds des Bildsensors auf Basis der Gruppe von Punktepaaren mit einem ersten Gewicht und den Bewegungsdaten mit einem zweiten Gewicht erfolgt. Zudem ist es möglich, die Plausibilität der Bewegungsdaten gegen die Gruppe von Punktepaaren zu prüfen und umgekehrt.

Bei einer weiteren bevorzugten Ausgestaltung weist das Berechnen der Messergebnisinformation ein erstes Berechnen von ersten Koordinaten des Realstartpunkts in einer Längeneinheit und ein zweites Berechnen von zweiten Koordinaten des Realendpunkts in der Längeneinheit auf und wird die Messergebnisinformation auf Basis der ersten und zweiten Koordinaten errechnet.

Die Berechnung der Koordinaten in einer Längeneinheit, also beispielsweise als (23mm,8mm,15mm), ermöglicht eine besonders einfache Weiterverarbeitung und Berechnung der Messergebnisinformation. Der Ursprung eines solchen Koordinatensystems wird hier, wie auch bei den anderen Ausgestaltungen, bevorzugt am distalen Ende des optische Visualisierungssystems bzw. Instruments mit mindestens einem Bildsensor der in der Mitte der Stereobasis gewählt.

Bei einer weiteren bevorzugten Ausgestaltung wird zumindest ein Bereich der vom Bildsensor erfassten Szene zumindest zeitweise mit einem Muster beleuchtet.

Die Beleuchtung mit einem Muster bzw. mit strukturiertem Licht ist insbesondere als Erweiterung zur Stereokonstruktion von Vorteil. Hierbei wird beispielsweise eine inhomogene, zeitlich variable Ausleuchtung einer endoskopischen Szene, in der die zu vermessende anatomische Struktur lokalisiert ist, vorgenommen. Ein wesentlicher Vorteil, der sich dadurch ergibt ist, dass damit auch in kontrastarmen Situationen eine verlässliche Stereokonstruktion möglich ist. Zeitlich lässt sich eine solche inhomogene Beleuchtung, beispielsweise unter Verwendung einer Matrix-LED, sehr schnell ansteuern (~ 1000 Hz), sodass durch entsprechende Auswahl der Betrachtungsmuster der Betrachter weiterhin einen homogenen Beleuchtungseindruck hat. Dabei ist es insbesondere vorteilhaft, wenn die Messvorrichtung eine Lichtquelle mit einer Vielzahl von individuell ansteuerbaren Einzellichtquellen zur Beleuchtung des Sichtfelds des Bildsensors und eine Steuereinheit aufweist, die ausgestaltet ist, die

Einzellichtquellen derart zu steuern, dass eine inhomogene und zeitliche variable Ausleuchtung des Sichtfelds des Bildsensors erreicht wird

Nach einem weiteren Aspekt wird die Aufgabe gelöst durch eine Messvorrichtung, aufweisend einen Bildsensor, vor dem ein optisches System angeordnet ist; und einen Prozessor, die dafür ausgebildet ist, die Schritte eines zuvor beschriebenen Messverfahrens auszuführen. Dabei steuert der Prozessor den Bildsensor zur Aufnahme von Bildern an. Optional kann der Prozessor auch eine Lichtquelle ansteuern.

Bei einer vorteilhaften Ausgestaltung weist die Messvorrichtung ferner eine Lichtquelle auf, die dafür ausgebildet ist, zumindest einen Bereich der vom Bildsensor erfassten Szene zumindest zeitweise mit einem Muster zu beleuchten.

Es versteht sich, dass eine bevorzugte Ausführungsform der Erfindung auch jede Kombination der abhängigen Ansprüche mit dem jeweiligen unabhängigen Anspruch sein kann. Eine Redundanz der abhängigen Ansprüche für die jeweiligen unabhängigen Ansprüche wird deshalb bewusst vermieden.

Es versteht sich ferner, dass das beanspruchte Messverfahren und das beanspruchte Messsystem ähnliche, entsprechende und/oder identische bevorzugte Ausführungsformen haben, insbesondere wie in den abhängigen Ansprüchen definiert und wie hierin offenbart.

Weitere vorteilhafte Ausführungsformen sind nachstehend definiert.

Die Erfindung wird nachfolgend anhand der Zeichnungen näher erläutert. Es zeigen:
Figur 1 eine schematische Darstellung einer Ausführungsform eines Messsystems mit einem Sichtfeld eines Bildsensors in einer ersten Lage;
Figur 2 die schematische Darstellung aus Figur 1 mit dem Sichtfeld des Bildsensors in einer zweiten Lage;
Figur 3 die schematische Darstellung aus Figur 1 mit dem Sichtfeld des Bildsensors in einer dritten Lage;
Figur 4 zwei Flussdiagramme zur schematischen Darstellung einer Ausführungsform eines Messverfahrens;
Figuren 5 und 6 Bildaufnahmen und entsprechende Tiefenkarten einer anatomischen Struktur;
Figur 7 eine weitere Bildaufnahme einer anatomischen Struktur;
Figur 8 eine Darstellung, wie der Bildsensor in unterschiedlichen Lagen seines Sichtfelds denselben Realpunkt erfasst;
Figur 9 eine schematische Darstellung der durchzuführenden Schritte zur Anzeige eines Abstands auf einer Anzeigevorrichtung gemäß einer Ausführungsform;
Figur 10 eine schematische Darstellung der Tiefenrekonstruktion basierend auf einem optischen Sensor und einem zusätzlichen Bewegungssensor gemäß einer Ausführungsform; und
Figuren 11 und 12 weitere Bildaufnahmen einer anatomischen Struktur.

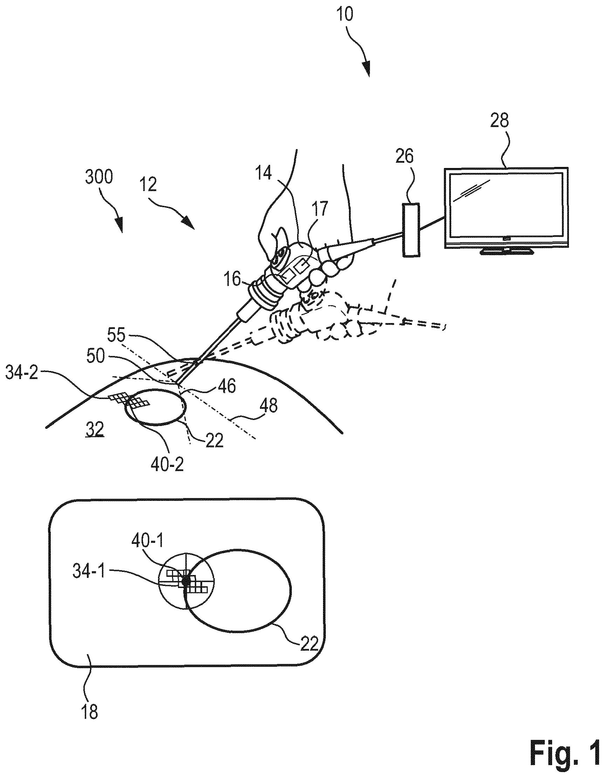

Figur 1 zeigt eine schematische Darstellung einer beispielhaften endoskopischen Messvorrichtung 10 in Verbindung mit einem Endoskop 12, an dem eine Kamera 14 mit einem symbolisch dargestellten Bildsensor 16 angeordnet ist. Der Bildsensor 16 weist zwei getrennte bildgebende Flächen auf, um ein linkes Bild und ein rechtes Bild für eine Stereoskopie zu erzeugen. Ortsfest zum Bildsensor 16 ist ein optionaler Bewegungssensor 17 angeordnet, der eine Verlagerung des Bildsensors 16 erfasst.

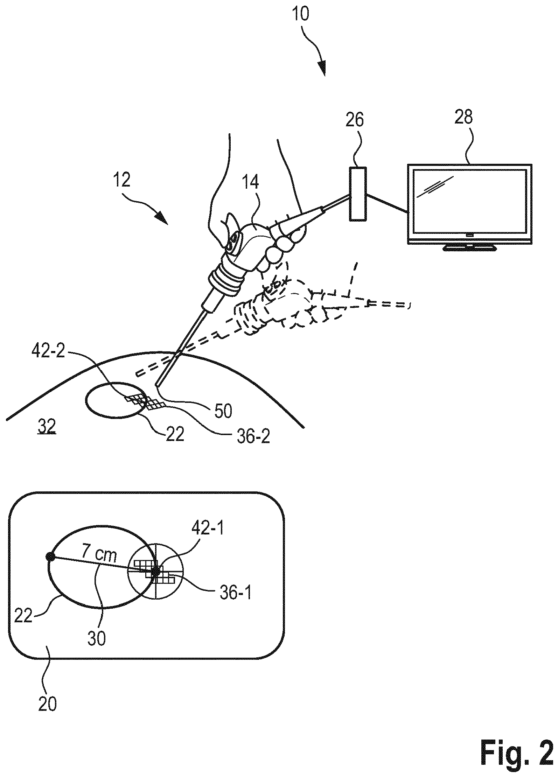

In Verbindung mit Figur 2 ist zu erkennen, dass ein erstes Bild 18 und ein zweites Bild 20 einer anatomischen Struktur 22 mithilfe des Bildsensors 16 aufgenommen werden. Der Bildsensor 16 ist vorzugsweise Element einer Videokamera. Das Endoskop 12 kann vorzugsweise mit einer Beleuchtungseinheit gekoppelt sein, die über Lichtleiter im Inneren des Endoskops 12 an einem distalen Ende 50 des Endoskops 12 eine zu untersuchende Szene 32 ausleuchtet. Zwischen dem Bildsensor 16 und dem distalen Ende 50 ist im Inneren des Endoskops 12 ein optisches System 55 angeordnet.

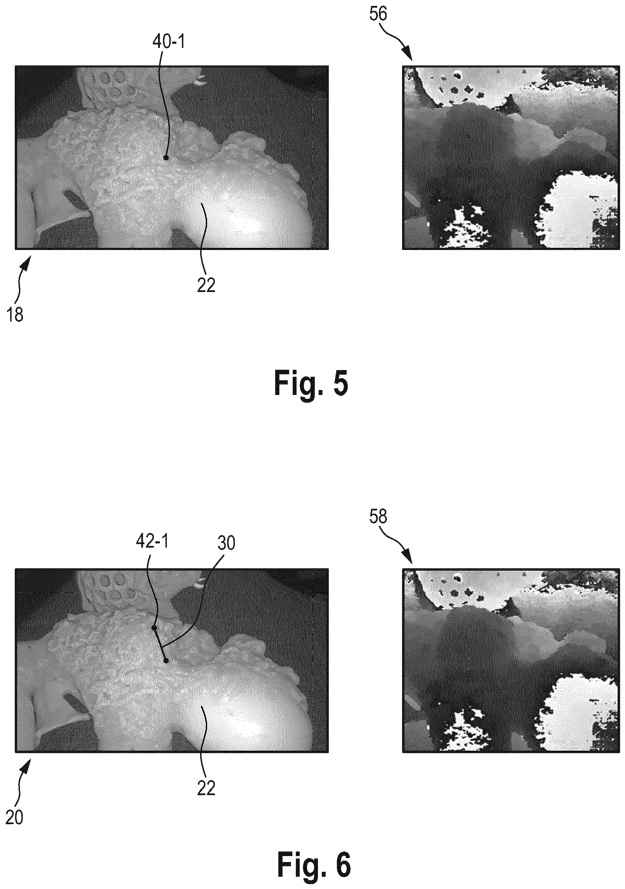

Die anatomische Struktur 22, welche in den aufgenommenen Bildern 18, 20 abgebildet wird, ist nur durch eine schematische ovale Darstellung gezeigt. Reale Aufnahmen einer zu untersuchenden anatomischen Struktur 22 sind z.B. in den Figuren 5, 7 und 11 gezeigt. Bei der zu untersuchenden Struktur 22 kann es sich beispielsweise um ein Aneurysma, einen Tumor oder eine sonstige anatomische Struktur handeln.

Durch einen Vergleich der Bilder 18 und 20 wird deutlich, dass das erste Bild 18 und das zweite Bild 20 aus unterschiedlichen Lagen aufgenommen wurden, denn die anatomische Struktur 22 ist im zweiten Bild 20 relativ zum ersten Bild 18 nach links verschoben. Dies bedeutet, dass sich die räumliche Lage, also der Abstand und/oder die Orientierung und/oder die Richtung, aus der der Bildsensor 16 Bilder der anatomischen Struktur 22 aufnimmt, zwischen der Aufnahme des ersten Bildes 18 und des zweiten Bildes 20 ändert. Diese Änderung kann beispielsweise manuell durch einen Benutzer, wie es in den Figuren 1 und 2 durch gestrichelte Linien gezeigt ist, oder durch einen Roboter erfolgen.

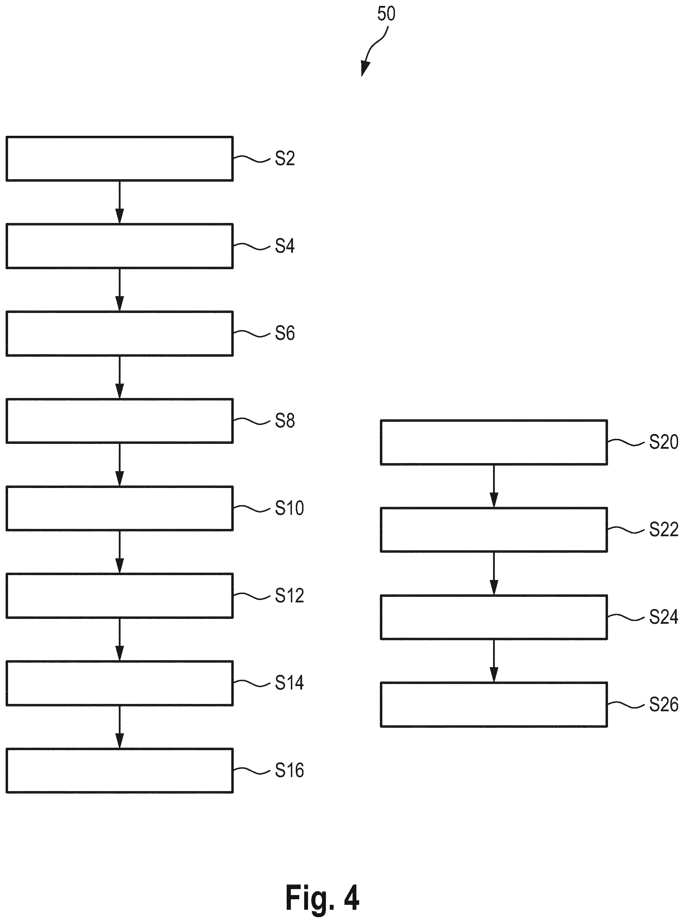

Die durch den Bildsensor 16 aufgenommenen Bilder 18, 20 werden an einen Prozessor 26 übergeben. Dieser ist vorzugsweise in einem Computer integriert und dafür ausgebildet, das erfindungsgemäße Messverfahren, siehe Figur 4, zur Berechnung der Messergebnisinformation, vorzugsweise eine Größenbestimmung der anatomischen Struktur 22, durchzuführen. Die Verarbeitung der aufgenommenen Bilder 18, 20 kann über eine Kamerakontrolleinheit (CCU) erfolgen. Ebenso möglich ist eine schnelle Verarbeitung über einen FPGA.

Nach der Verarbeitung der aufgenommenen Bilder 18, 20 durch den Prozessor 26 werden die Bilder, wie in der Endoskopie üblich, auf einer Anzeigevorrichtung 28 angezeigt. Die Anzeige erfolgt bevorzugt in Kombination mit einer Messergebnisinformation 30, welche von dem Prozessor 26 zuvor berechnet wurde. Bei dieser Messergebnisinformation 30 kann es sich, wie beispielhaft in Figur 2 gezeigt ist, um die Länge der anatomischen Struktur 10 handeln, hier 7 cm.

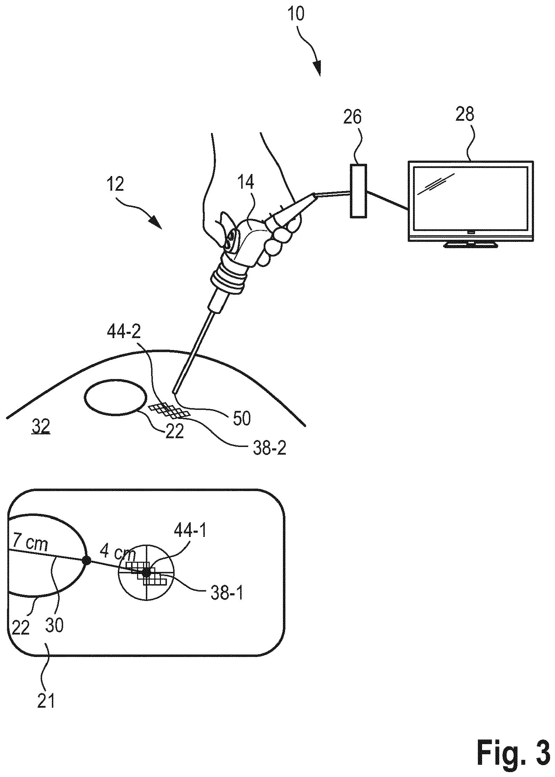

Figur 4 zeigt in der Zusammenschau mit den Figuren 1 bis 3 ein Ausführungsbeispiel eines Messverfahrens 50. Im Schritt S2 wird ein erstes Bild von einer Szene 32 aufgenommen. Dieses Aufnehmen S2 erfolgt mit dem Bildsensor 16, vor dem das optische System 55 angeordnet ist, wobei in dem ersten Bild 18 eine erste Vielzahl von symbolisch dargestellten ersten Bildpunkten 34-1 eine entsprechende erste Vielzahl von symbolisch dargestellten ersten Realpunkten 34-2 der Szene 32 mittels des Bildsensors 16 abbildet. Ein symbolisch dargestellter Bildstartpunkt 40-1 der ersten Vielzahl von Bildpunkten 34-1 definiert einen Realstartpunkt 40-2 in der Szene 32, wobei sich ein Sichtfeld 46 (durch zwei gestrichelte Linien angedeutet) des Bildsensors 16 beim Aufnehmen des ersten Bildes 18 in einer ersten räumlichen Lage relativ zu der Szene 32 befindet.

Im Schritt S4 werden erste Tiefeninformationen ermittelt, die zumindest für eine erste Teilmenge der ersten Vielzahl von ersten Bildpunkten 34-1, einschließlich des Bildstartpunkts 40-1, einen jeweiligen Abstand eines von einem ersten Bildpunkt 34-1 abgebildeten ersten Realpunkts 34-2 der ersten Vielzahl von ersten Realpunkten 34-2 zu einer Referenzebene 48 (symbolisch mit einer strichgepunkteten Linie dargestellt) beschreiben.

Im Schritt S6 wird ein zweites Bild 20 der Szene 32 aufgenommen, wobei das Aufnehmen S6 mit dem Bildsensor 16 erfolgt. In dem zweiten Bild 20 bildet eine zweite Vielzahl von symbolisch dargestellten zweiten Bildpunkten 36-1 eine entsprechende zweite Vielzahl von symbolisch dargestellten zweiten Realpunkten 36-2 der Szene 32 mittels des Bildsensors 16 ab. Ein Bildendpunkt 42-1 der zweiten Vielzahl von zweiten Bildpunkten 36-1 definiert einen Realendpunkt 36-2 in der Szene 32. Das Sichtfeld 46 des Bildsensors 16 befindet sich beim Aufnehmen des zweiten Bildes 20 in einer zweiten räumlichen Lage relativ zu der Szene 32, die sich von der ersten räumlichen Lage unterscheidet.

Im Schritt S8 werden zweite Tiefeninformationen ermittelt, die zumindest für eine zweite Teilmenge der zweiten Vielzahl von zweiten Bildpunkten 36-1, einschließlich des Bildendpunkts 42-1, einen jeweiligen Abstand eines vom zweiten Bildpunkt 36-1 abgebildeten zweiten Realpunkts 36-2 der zweiten Vielzahl von zweiten Realpunkten 36-2 zu der Referenzebene 48 beschreiben.

Im Schritt S10 wird eine Gruppe von Punktepaaren bestimmt, die jeweils einen ersten Bildpunkt 34-1 aus der ersten Vielzahl von ersten Bildpunkten 34-1 und einen zweiten Bildpunkt 36-1 aus der zweiten Vielzahl von zweiten Bildpunkten 36-1 enthalten, die miteinander derart korrespondieren, dass der erste und der zweite Bildpunkt 34-1, 36-1 eines Punktepaars einen selben Realpunkt 34-2 abbilden.

Im Schritt S12 wird eine räumliche Lageänderung des Sichtfelds 46 des Bildsensors 16 auf Basis der Gruppe von Punktepaaren bestimmt.

Im Schritt S14 wird eine Messergebnisinformation 30 basierend auf einer ersten Position des Bildstartpunkts 34-1 auf dem Bildsensor 16, einer zweiten Position des Bildendpunkts 36-1 auf dem Bildsensor 16, einer Tiefeninformation für den Bildstartpunkt 40-1 und/oder den Bildendpunkt 42-1, den Abbildungseigenschaften des optischen Systems 55 und der räumlichen Lageänderung des Sichtfelds 46 berechnet, wobei die Messergebnisinformation 30 eine Länge oder eine Fläche in der Szene 32 ist.

Schließlich wird im Schritt S16 die Messergebnisinformation 30 ausgegeben, insbesondere auf der Anzeigevorrichtung 28.

Das Messverfahren 50 kann weitere Schritte aufweisen, die dem Schritt S12 folgen können, also bevor die Messergebnisinformation 30 berechnet S14 und ausgeben S16 wird, oder dem Schritt S16 folgen können, wobei dann nach dem Berechnen S14 und Ausgeben S16 der Messergebnisinformation 30 eine modifizierte oder erweiterte Messergebnisinformation berechnet und ausgeben wird.

Im Schritt S20 wird ein drittes Bild 21 der Szene 32 aufgenommen, wobei das Aufnehmen S20 mit dem Bildsensor 16 erfolgt. In dem dritten Bild 21 bildet eine dritte Vielzahl von dritten Bildpunkten 38-1 eine entsprechende dritte Vielzahl von dritten Realpunkten 38-2 der Szene 32 mittels des Bildsensors 16 ab. Ein Bildfolgepunkt 44-1 der dritten Vielzahl von dritten Bildpunkten definiert einen Realfolgepunkt 44-2 in der Szene 32, wobei sich das Sichtfeld 46 des Bildsensors 16 beim Aufnehmen des dritten Bildes 21 in einer dritten räumlichen Lage relativ zu der Szene 32 befindet, die sich von der ersten und der zweiten räumlichen Lage unterscheidet.

Im Schritt S22 werden dritte Tiefeninformationen ermittelt, die zumindest für eine dritte Teilmenge der dritten Vielzahl von dritten Bildpunkten 38-1, einschließlich des Bildfolgepunkts 44-1, einen jeweiligen Abstand eines von einem dritten Bildpunkt 38-1 abgebildeten dritten Realpunkts 38-2 der dritten Vielzahl von dritten Realpunkten 38-2 zu der Referenzebene 48 beschreiben.

Im Schritt S24 wird eine weitere Gruppe von Punktepaaren bestimmt, die jeweils einen zweiten Bildpunkt 36-1 aus der zweiten Vielzahl von zweiten Bildpunkten 36-1 und einen dritten Bildpunkt 38-1 aus der dritten Vielzahl von dritten Bildpunkten 38-1 enthalten, die miteinander derart korrespondieren, dass der zweite und der dritte Bildpunkt 36-1, 38-1 eines Punktepaars einen selben Realpunkt 36-2 abbilden.

Im Schritt S26 wird eine weitere räumliche Lageänderung des Sichtfelds 46 des Bildsensors 16 auf Basis der weiteren Gruppe von Punktepaaren bestimmt.

Das Berechnen, Schritt S14, der Messergebnisinformation 30 kann nun ferner auf einer dritten Position des Bildfolgepunkts 44-1 auf dem Bildsensor 16, den dritten Tiefeninformationen und der weiteren räumlichen Lageänderung des Sichtfelds 46 basieren, oder es wird eine weitere Messergebnisinformation 30 basierend auf einer dritten Position des Bildfolgepunkts 44-1 auf dem Bildsensor 16, den dritten Tiefeninformationen und der weiteren räumlichen Lageänderung des Sichtfelds 46 berechnet.

Figuren 5 und 6 zeigen Bildaufnahmen und entsprechende Tiefenkarten 56, 58 einer anatomischen Struktur 22. Die Tiefenkarten 56, 58 sind Beispiele der Tiefeninformationen 40 der vorliegenden Erfindung.

Figur 5 zeigt ein erstes Bild 18 der anatomischen Struktur 22 (links) und eine dazugehörige ermittelte Tiefenkarte 56 (rechts). Figur 6 zeigt ein zweites Bild 20 derselben anatomischen Struktur 22 (links) und eine dazugehörige ermittelte Tiefenkarte 58 (rechts). Wie in den Figuren 1 und 2 gezeigt ist, werden die Bilder 18, 20 beispielsweise mit dem Bildsensor 16 eines Endoskops 12 aufgenommen und dieses Endoskop 12 wird manuell oder mithilfe eines Roboters zwischen der Aufnahme des ersten Bildes 18 und des zweiten Bildes 20 translatorisch und/oder rotatorisch bewegt. Demnach wird die anatomische Struktur 22 in den beiden Bildern 18, 20 aus unterschiedlichen Lagen betrachtet. Dies hat zur Folge, dass ein anderes Merkmal der anatomischen Struktur 10 in den Bildmitten des ersten Bildes 18 und des zweiten Bildes 20 zu sehen ist.

Die entsprechenden Tiefenkarten 56, 58 können beispielsweise über die Disparität zwischen den beiden Bildern der Bildsensoren eines Stereoendoskops bzw. zwischen den Bildsensoren eines Stereoendoskops oder unter Zuhilfenahme eines Tiefenbildsensors, wie einer Laufzeitkamera (Time-of-Flight-Kamera), ermittelt werden. Mithilfe der Tiefenkarten 56, 58 ist es möglich, 3D-Koordinatenpunkte zu bestimmen, für die aufgrund der ermittelten Tiefeninformationen die x-, y- als auch die z-Koordinaten bekannt sind. Wie bereits erwähnt können die beiden Bilder auch durch Pseudostereoskopie gewonnen werden.

Im ersten Bild 18 erfolgt die Bestimmung des Realstartpunkts 40-2 demnach durch die Auswahl des Bildstartpunkts 40-1, hier im Mittelpunkt des ersten Bildes 18 und die Ermittlung der z-Koordinate über die entsprechende Tiefenkarte 56. Im zweiten Bild 20 erfolgt die Bestimmung des Realendpunkts 42-2 durch die entsprechende Auswahl des Bildendpunkts 42-1 und die Ermittlung der z-Koordinate über die entsprechende Tiefenkarte 58. Es sei angemerkt, dass dies nur beispielhaft ist. Die Ermittlung von vollständigen Tiefenkarten ist grundsätzlich nicht nötig, da die Tiefeninformationen zum Bildstartpunkt 40-1 und zum Bildendpunkt 42-1 genügen, um den realen Abstand zweier Merkmale der anatomischen Struktur 22 zu bestimmen.

Die aufgenommenen Bilder 18, 20 sind vorzugsweise Full HD-Bilder mit einer Auflösung mit 1920 x 1080 Pixel oder 4K-Bilder mit einer Auflösung mit 3840 x 2160 Pixel. Dabei kann es vorteilhaft sein, wenn jeweils der Realstartpunkt 40-2 und der Realendpunkt 42-2 - nach einer Verlagerung des Endoskops 12 - in der Bildmitte an der Pixelposition (960, 540) ausgewählt werden.

Nach dem Bestimmen des ersten 3D-Koordinaten für den Realstartpunkt 40-2 und den Realendpunkt 42-2 kann eine Messergebnisinformation 80, wie beispielsweise der Abstand zwischen Realstartpunkt 40-2 und Realendpunkt 42-2 berechnet werden. Wie in Figur 6 zu sehen ist, wird dieser berechnete Abstand anschließend im zweiten Bild 20 angezeigt. Vorzugsweise sind die Bilder 18, 20 einzelne Frames eines Videos, welches beispielweise mit einem Videoendoskop während einer medizinischen Untersuchung aufgenommen wird.

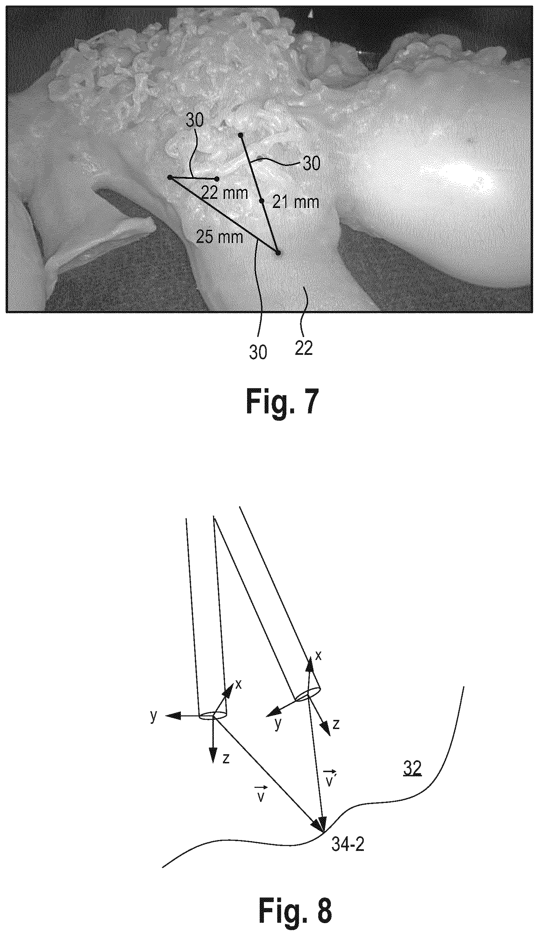

Figur 7 zeigt eine weitere Bildaufnahme einer anatomischen Struktur 22 nach Anwendung des erfindungsgemäßen Messverfahrens. Es ist zu sehen, dass durch das erfindungsgemäße Messverfahren mehrere Abstände (10 mm, 21 mm und 25 mm) zwischen unterschiedlichen Merkmalen der anatomischen Struktur 22 über die Bildabfolge mehrerer Bilder berechnet und angezeigt werden können. Das medizinische Fachpersonal ist somit in der Lage, die Dimension der anatomischen Struktur 22 verlässlich über unterschiedliche Abstandsmessungen zu bestimmen. Optional können Unsicherheiten in den ermittelten Abständen mit einer Standardabweichung angegeben werden.

Figur 8 verdeutlicht, wie derselbe Realpunkt 34-2 von einem Bildsensor 16 in unterschiedlichen Lagen an unterschiedlichen Positionen erfasst wird. Das xyz-Bezugssystem bleibt konstant, auch die Referenzebene 48, die hier die xy-Ebene ist. In der ersten räumlichen Lage, links, erscheint der Realpunkt 34-2 mit den Koordinaten eines Vektors *̅v̅*̅. In der zweiten räumlichen Lage, rechts, erscheint der Realpunkt 34-2 mit den Koordinaten eines Vektors *̅v̅'̅*̅. Derselbe Realpunkt 34-2 erscheint also aus der Sicht des Bildsensors 14 in Verbindung mit der Tiefeninformation an zwei verschiedenen Koordinaten. Indem genügend Punktepaare von Bildpunkten 34-1 ermittelt werden, die jeweils einen selben Realpunkt 34-2 abbilden, kann die Lageänderung des Sichtfelds 46 des Bildsensors 16 bestimmt werden.

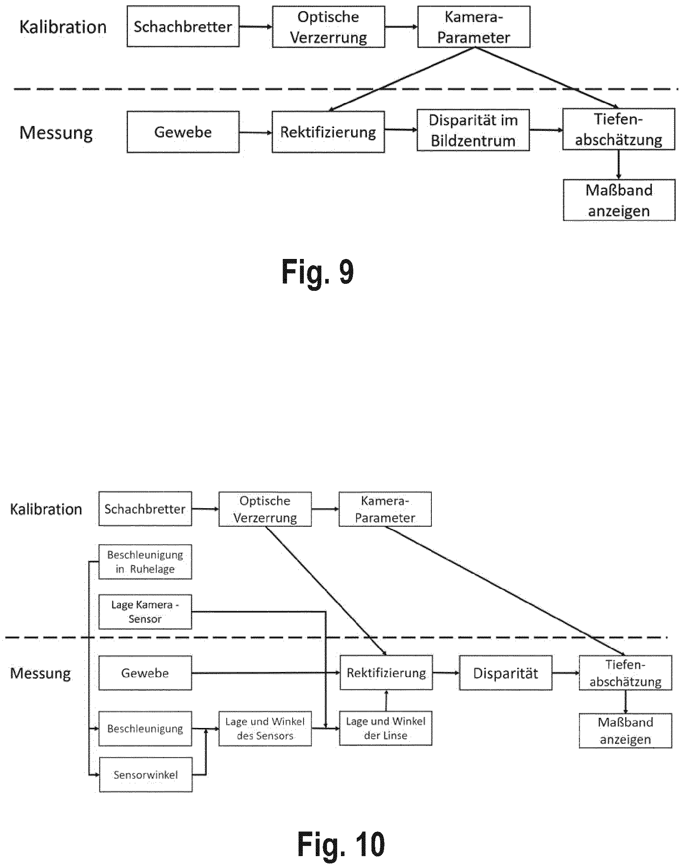

Fig. 9 zeigt bei einer bevorzugten Ausführungsform eine schematische Darstellung der durchzuführenden Schritte zur Anzeige eines Abstands als Messinformation 30 in Form eines Maßbands auf einer Anzeigevorrichtung 28. Es findet zunächst eine Kalibration des Bildsensors 16 statt, welcher beispielsweise Teil einer Videokamera eines Videoendoskops ist. Über die Darstellung von Schachbrettern wird eine mögliche optische Verzerrung in den aufgenommenen Bildern des Bildsensors 16 ermittelt. Im nächsten Kalibrierprozess werden sodann die Kameraparameter bestimmt.

Die Messung startet mit der Aufnahme von Bildern des Gewebes bzw. der anatomischen Struktur 22. Die während des Kalibrierprozesses bestimmten Kameraparameter können für eine anschließende Rektifizierung verwendet werden, bei der geometrische Verzerrungen in den Bilddaten, welche beispielsweise durch eine falsche Orientierung der Kamera entstehen, eliminiert werden können. Anschließend wird basierend auf der Disparität im Bildzentrum der aufgenommenen Bilder die Tiefe abgeschätzt. Sind die entsprechenden Tiefeninformationen bekannt, kann abschließend der Abstand bestimmt werden und ein entsprechendes Maßband im Bild angezeigt werden.

Fig. 10 zeigt eine schematische Darstellung der Tiefenrekonstruktion basierend auf der Kamera und einem zusätzlichen Bewegungssensor. Der Bewegungssensor ist beispielsweise ein Beschleunigungssensor, welcher in der Kamera integriert sein kann. Der Beschleunigungssensor registriert die Bewegungen der Kamera und durch zweifache Integration der Beschleunigung erhält man einen quantitativen Wert für die Bewegung der Kamera.

Im Gegensatz zu Figur 9 sind in Figur 10 noch weitere Verfahrensschritte gezeigt. So werden über die Beschleunigung des Bewegungssensors in Ruhelage (durch die Gravitation) und die Beschleunigung und den Sensorwinkel (gegenüber der Gravitationsachse) des Beschleunigungssensors während der Bewegung Informationen zur Lage und Winkel der optischen Linse der Kamera ermittelt. Diese Informationen können anschließend für eine genauere Rektifizierung verwendet werden.

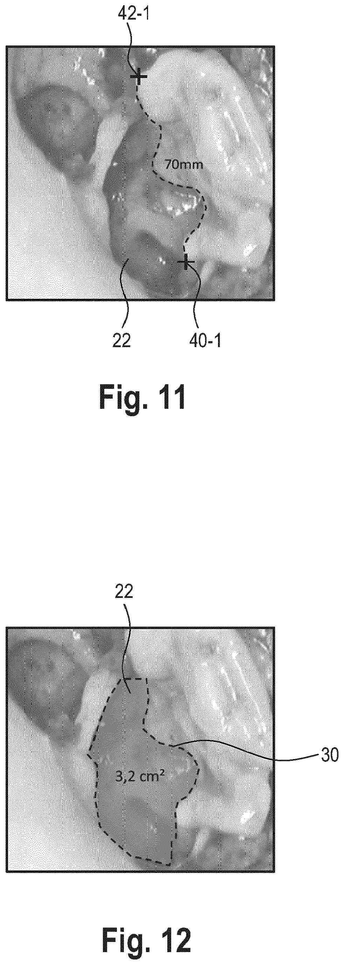

Figuren 11 und 12 zeigen weitere Bildaufnahmen einer anatomischen Struktur 22 und eine Konturbestimmung entlang der Grenze der anatomischen Struktur 22. Dies ist insbesondere dann möglich, wenn die Translation und Rotation des Sichtfelds des Bildsensors 16 bekannt ist. Dies kann, wie beispielsweise in Fig. 10 gezeigt ist, durch die Messung mit einem Beschleunigungssensor, welcher mit dem Bildsensor 16 mechanisch gekoppelt ist, erfolgen.

Demnach können zwischen dem Bildstartpunkt 40-1 und dem Bildendpunkt 42-1 nicht nur Messergebnisinformationen 30 in Form eines Abstands, d.h. der kürzesten Strecke zwischen den beiden Koordinatenpunkten, berechnet werden, sondern ebenso Messergebnisinformationen 30 in Form einer krummlinigen Kontur.

Durch die ermittelte Translation des Bildsensors 16 zwischen der Aufnahme von unterschiedlichen Bildern einer Videosequenz ist es möglich nachzuverfolgen, entlang welches Pfads der Bildsensor 16 während der medizinischen Untersuchung bewegt wurde. Dies kann beispielsweise anhand von mehreren Bild- bzw. Realfolgepunkten 44-1, 44-2 erfolgen.

In Figur 11 ist gezeigt, dass dadurch beispielsweise eine krummlinige Begrenzung einer anatomischen Struktur 22 vermessen werden kann. In Figur 12 ist gezeigt, dass selbst eine Messung des Flächeninhalts oder des Umfangs der anatomischen Struktur 22 möglich ist.

Insgesamt wird demnach durch das vorgestellte Messverfahren und das entsprechende Messsystem eine einfache und vielseitige Messfunktion zur Größenbestimmung einer anatomischen Struktur bereitgestellt.

Es versteht sich, dass die gezeigten Ausführungsformen lediglich beispielhaft zu verstehen sind und dass weitere Variationen möglich sind ohne den Erfindungsgedanken zu verlassen. Die gezeigten Ausführungsformen sollen demnach nicht als schutzeinschränkend verstanden werden.

## Patentansprüche

1. Messverfahren (50), aufweisend die folgenden Schritte:
Aufnehmen (S2) eines ersten Bildes (18) von einer Szene (32), wobei das Aufnehmen (S2) mit einem Bildsensor (16), vor dem ein optisches System (55) angeordnet ist, erfolgt, wobei in dem ersten Bild (18) eine erste Vielzahl von ersten Bildpunkten (34-1) eine entsprechende erste Vielzahl von ersten Realpunkten (34-2) der Szene (32) mittels des Bildsensors (16) abbildet und wobei ein Bildstartpunkt (40-1) der ersten Vielzahl von ersten Bildpunkten (34-1) einen Realstartpunkt (40-2) in der Szene (32) definiert, wobei sich ein Sichtfeld (46) des Bildsensors (16) beim Aufnehmen (S2) des ersten Bildes (18) in einer ersten räumlichen Lage relativ zu der Szene (32) befindet;
Ermitteln (S4) von ersten Tiefeninformationen, die zumindest für eine erste Teilmenge der ersten Vielzahl von ersten Bildpunkten (34-1), einschließlich des Bildstartpunkts (40-1), einen jeweiligen Abstand eines von einem ersten Bildpunkt (34-1) abgebildeten ersten Realpunkts (34-2) der ersten Vielzahl von ersten Realpunkten (34-2) zu einer Referenzebene (48) beschreiben;
Aufnehmen (S6) eines zweiten Bildes (20) der Szene (32), wobei das Aufnehmen (S6) mit dem Bildsensor (16) erfolgt, wobei in dem zweiten Bild (20) eine zweite Vielzahl von zweiten Bildpunkten (36-1) eine entsprechende zweite Vielzahl von zweiten Realpunkten (36-2) der Szene (32) mittels des Bildsensors (16) abbildet und wobei ein Bildendpunkt (42-1) der zweiten Vielzahl von zweiten Bildpunkten einen Realendpunkt (42-2) in der Szene (32) definiert, wobei sich das Sichtfeld (46) des Bildsensors (16) beim Aufnehmen (S6) des zweiten Bildes in einer zweiten räumlichen Lage relativ zu der Szene (32) befindet, die sich von der ersten räumlichen Lage unterscheidet;
Ermitteln (S8) von zweiten Tiefeninformationen, die zumindest für eine zweite Teilmenge der zweiten Vielzahl von zweiten Bildpunkten (36-1), einschließlich des Bildendpunkts (42-1), einen jeweiligen Abstand eines von einem zweiten Bildpunkt (36-1) abgebildeten zweiten Realpunkts (36-2) der zweiten Vielzahl von zweiten Realpunkten (36-2) zu der Referenzebene (48) beschreiben;
Bestimmen (S10) einer Gruppe von Punktepaaren, die jeweils einen ersten Bildpunkt (34-1) aus der ersten Vielzahl von ersten Bildpunkten (34-1) und einen zweiten Bildpunkt (36-1) aus der zweiten Vielzahl von zweiten Bildpunkten (36-1) enthalten, die miteinander derart korrespondieren, dass der erste und der zweite Bildpunkt (34-1, 36-1) eines Punktepaars einen selben Realpunkt (34-2) abbilden;
Bestimmen (S12) einer räumlichen Lageänderung des Sichtfelds (46) des Bildsensors (16) auf Basis der Gruppe von Punktepaaren;
Berechnen (S14) von Messergebnisinformation (30) basierend auf einer ersten Position des Bildstartpunkts (40-1) auf dem Bildsensor (16), einer zweiten Position des Bildendpunkts (42-1) auf dem Bildsensor (16), einer Tiefeninformation für den Bildstartpunkt (40-1) und/oder den Bildendpunkt (42-1), den Abbildungseigenschaften des optischen Systems (55) und der räumlichen Lageänderung des Sichtfelds (46), wobei die Messergebnisinformation (30) eine Länge oder eine Fläche in der Szene (32) ist; und
Ausgeben (S16) der Messergebnisinformation (30).

2. Messverfahren (50) nach Anspruch 1, ferner mit den Schritten:
Aufnehmen (S20) eines dritten Bildes (21) der Szene (32), wobei das Aufnehmen (S20) mit dem Bildsensor (16) erfolgt, in dem dritten Bild (21) eine dritte Vielzahl von dritten Bildpunkten (38-1) eine entsprechende dritte Vielzahl von dritten Realpunkten (38-2) der Szene (32) mittels des Bildsensors (16) abbildet und ein Bildfolgepunkt (44-1) der dritten Vielzahl von dritten Bildpunkten (38-1) einen Realfolgepunkt (44-2) in der Szene (32) definiert, wobei sich das Sichtfeld (46) des Bildsensors (16) beim Aufnehmen des dritten Bildes (21) in einer dritten räumlichen Lage relativ zu der Szene (32) befindet, die sich von der ersten und der zweiten räumlichen Lage unterscheidet;
Ermitteln (S22) von dritten Tiefeninformationen, die zumindest für eine dritte Teilmenge der dritten Vielzahl von dritten Bildpunkten (38-1), einschließlich des Bildfolgepunkts (44-1), einen jeweiligen Abstand eines von einem dritten Bildpunkt (38-1) abgebildeten dritten Realpunkts (38-2) der dritten Vielzahl von dritten Realpunkten (38-2) zu der Referenzebene (48) beschreiben;
Bestimmen (S24) einer weiteren Gruppe von Punktepaaren, die jeweils einen zweiten Bildpunkt (36-1) aus der zweiten Vielzahl von zweiten Bildpunkten (36-1) und einen dritten Bildpunkt (38-1) aus der dritten Vielzahl von dritten Bildpunkten (38-1) enthalten, die miteinander derart korrespondieren, dass der zweite und der dritte Bildpunkt (36-1, 38-1) eines Punktepaars einen selben Realpunkt (36-2) abbilden;
Bestimmen (S26) einer weiteren räumlichen Lageänderung des Sichtfelds des Bildsensors auf Basis der weiteren Gruppe von Punktepaaren;
wobei das Berechnen (S14) der Messergebnisinformation (30) ferner auf einer dritten Position des Bildfolgepunkts (44-1) auf dem Bildsensor (16) und der weiteren räumlichen Lageänderung des Sichtfelds (46) basiert oder eine weitere Messergebnisinformation basierend auf einer dritten Position des Bildfolgepunkts (44-1) auf dem Bildsensor (16) und der weiteren räumlichen Lageänderung des Sichtfelds (46) berechnet wird.

3. Messverfahren (50) nach einem der vorhergehenden Ansprüche, wobei nach dem Aufnehmen (S2) des ersten Bildes (18) und vor dem Aufnehmen (S6; S20) des zweiten oder des dritten Bildes (20; 21) folgende Schritte ausgeführt werden:
Aufnehmen mindestens eines Zwischenbilds, so dass eine Sequenz beginnend mit dem ersten Bild (18), fortgeführt über das mindestens eine Zwischenbild und endend mit dem zweiten oder dem dritten Bild (20; 21) gebildet wird, wobei in jedem Zwischenbild eine weitere Vielzahl von weiteren Bildpunkten eine entsprechende weitere Vielzahl von weiteren Realpunkten der Szene (32) mittels des Bildsensors (16) abbildet und wobei sich das Sichtfeld (46) des Bildsensors (16) beim Aufnehmen des Zwischenbildes in einer weiteren räumlichen Lage relativ zu der Szene (32) befindet, die sich von der innerhalb der Sequenz vorherigen räumlichen Lage unterscheidet;
nach dem Aufnehmen eines Zwischenbilds von dem mindestens einen Zwischenbild:
Ermitteln von weiteren Tiefeninformationen, die zumindest für eine weitere Teilmenge der weiteren Vielzahl von weiteren Bildpunkten einen jeweiligen Abstand eines von einem weiteren Bildpunkt abgebildeten weiteren Realpunkts der weiteren Vielzahl von weiteren Realpunkten zu der Referenzebene (48) beschreiben;
Bestimmen einer Zwischengruppe von Punktepaaren, die jeweils einen ersten Sequenzbildpunkt aus der weiteren Vielzahl von weiteren Bildpunkten in dem Zwischenbild und einen zweiten Sequenzbildpunkt in einem vorherigen Bild aus der Sequenz enthalten, die miteinander derart korrespondieren, dass der erste und der zweite Sequenzbildpunkt eines Punktepaars einen selben Realpunkt (34-2) abbilden;
wobei das Bestimmen (S12; S26) der räumlichen Lageänderung oder der weiteren räumlichen Lageänderung zudem auf Basis der mindestens einen weiteren Zwischengruppe von Punktepaaren erfolgt.

4. Messverfahren (50) nach einem der vorhergehenden Ansprüche, wobei zumindest ein Teil der ersten und zweiten Tiefeninformationen aus einer Stereobildinformation gewonnen wird.

5. Messverfahren nach einem der vorhergehenden Ansprüche, wobei die Messergebnisinformation (30) einen Abstand zwischen dem Realstartpunkt (34-2) und dem Realendpunkt (36-2) aufweist.

6. Messverfahren nach einem der vorhergehenden Ansprüche, wobei die Messergebnisinformation (30) die Länge einer Strecke entlang einer Kontur zwischen dem Realstartpunkt (34-2) und dem Realendpunkt (36-2) aufweist.

7. Messverfahren nach einem der vorhergehenden Ansprüche, wobei die Messergebnisinformation (30) den Inhalt einer Fläche aufweist, deren Begrenzung unter Berücksichtigung des Realstartpunkts (34-2) und des Realendpunkts (36-2) ermittelt wird.

8. Messverfahren nach einem der vorhergehenden Ansprüche, wobei zumindest ein Teil der ersten und zweiten Tiefeninformationen aus einer Laufzeitinformation gewonnen wird, die der Bildsensor (16) oder ein Tiefenbildsensor bereitstellt.

9. Messverfahren nach einem der vorhergehenden Ansprüche, wobei zumindest ein Teil der ersten und zweiten Tiefeninformationen aus einer Fokusinformation des optischen Systems (55) gewonnen wird.

10. Messverfahren nach einem der vorhergehenden Ansprüche, wobei das Definieren des Realstartpunktes (40-2) und des Realendpunktes (42-2) durch eine manuelle Benutzeraktion erfolgt.

11. Messverfahren nach einem der vorhergehenden Ansprüche, wobei die räumliche Lageänderung des Sichtfelds (46) des Bildsensors (16) unter Berücksichtigung von Bewegungsdaten bestimmt wird, die von einem Bewegungssensor (17) aufgenommen werden, der relativ zum Bildsensor (16) ortsfest angeordnet ist.

12. Messverfahren nach einem der vorhergehenden Ansprüche, wobei das Berechnen (S16) der Messergebnisinformation (30) ein erstes Berechnen von ersten Koordinaten des Realstartpunkts (34-2) in einer Längeneinheit und ein zweites Berechnen von zweiten Koordinaten des Realendpunkts (36-2) in der Längeneinheit aufweist und wobei die Messergebnisinformation (30) auf Basis der ersten und zweiten Koordinaten errechnet wird.

13. Messverfahren (50) nach einem der vorhergehenden Ansprüche, wobei zumindest ein Bereich der vom Bildsensor (16) erfassten Szene (32) zumindest zeitweise mit einem Muster beleuchtet wird.

14. Messvorrichtung (10), aufweisend:
einen Bildsensor (16), vor dem ein optisches System (55) angeordnet ist; und
einen Prozessor (26), der dafür ausgebildet ist, die Schritte eines Messverfahrens einem der Ansprüche 1 bis 11 auszuführen.

15. Messvorrichtung nach Anspruch 14, ferner mit einer Lichtquelle, die dafür ausgebildet ist, zumindest einen Bereich der vom Bildsensor (16) erfassten Szene (32) zumindest zeitweise mit einem Muster zu beleuchten.
